# EUROPEAN PATENT APPLICATION

(11) **EP 2 177 218 A1**
(43) Date of publication of application: **21.04.2010**
(21) Application number: 08450158.4
(22) Date of filing: 15.10.2008
(51) Int. Cl.: A61K 31/4015, A61K 31/407, A61K 31/4409, A61K 31/551, A61K 31/7076, A61K 31/7105, A61K 39/395, A61P 25/00, A61P 25/02, A61P 25/28

(54) **Regenerative therapy**

(71) Applicant: Medizinische Universität Wien, 1090 Vienna (AT)
(72) Inventor: Baer, Alexandra, 1090 Vienna (AT); Kotter, Mark, CB17XA Cambridge (GB)
(74) Representative: Sonn, Helmut

(57) **Abstract**

The present invention relates to the use of an agent capable of enhancing the PKA-p38-CREB pathway, suppressing the Fyn-RhoA-Rock and/or PKC-alpha-MARCKS pathways or Ephrin-B3 for the manufacture of a pharmaceutical composition for the treatment of damaged myelin sheaths, **characterized in that**, the agent neutralizes the effect of inhibitors of the regeneration of damaged myelin sheaths by oligodendrocytes, and wherein the inhibitors are selected from myelin associated inhibitors, in particular Ephrin-B3, and inhibitors of the glial scar, in particular Sema3A, and kits suitable for the treatment of damaged myelin sheaths.

## Description

The present invention relates to methods for the treatment of damaged myelin sheaths, in particular by remyelination, and suitable pharmaceutical agents for the treatment.

Cell populations in the adult central nervous system (CNS) exist which are able to give rise to cells of all major cell lineages including neurons, oligodendrocytes, and astrocytes. Although the ability of these cells to replace lost neurons is relatively poor, their ability to replace oligodendrocytes (and hence remyelinate demyelinated axons) can be quite efficient. Remyelination in the CNS is mediated by a multipotent adult stem / precursor cell population traditionally referred to as oligodendrocyte precursor cells (OPCs). Although remyelination can be efficient, it often fails in clinical disease for reasons that are not fully understood. The chronic demyelination that follows is associated with axonal loss of function, so providing a possible mechanism for progressive disability in patients suffering from diseases in which chronic demyelination occurs, of which the most important is multiple sclerosis. Failure of OPC differentiation is a major cause of remyelination failure.

A possible explanation for the failure of OPC differentiation in MS is the presence of inhibitors within demyelinated lesions. A number of potential inhibitors have been proposed including axonal PSA-NCAM in chronically demyelinated lesions, inhibitors that form part of the glial scar such as astrocytic hyaluronan and notch-jagged signalling. Recently it has also been shown that myelin debris, accumulating in acute lesions as a result of demyelination, exerts a powerful inhibitory effect on OPC differentiation and needs to be cleared by phagocytes before remyelination can occur (Kotter et al., J. Neurosci 26 (2006): 328-332). With ageing the efficiency of myelin debris removal decreases, and this may contribute to the impairment in OPC differentiation that underlies the age-associated decline in remyelination efficiency.
Axon remyelination is distinct from axon regeneration in general. Axon regeneration is the process of promoting the reformation of destroyed or severed neuronal axons. Axons are slender projections of a nerve cell or neuron that conduct electrical impulses which are transmitted from the neuron's cell body to recipient cells via synapses. Axons, or nerve fibers themselves are microscopic in diameter but may be significantly longer extending to a meter or even longer. Axons of many neurons are sheathed in myelin, which is a lipid rich membrane structure performing a protection and signal accelerating function. Myelin is made up primarily of a glycolipid called galactocerebroside. The intertwining of the hydrocarbon chains of sphingomyelin serve to strengthen the myelin sheath.

Demyelination is the act of loss of the myelin sheath insulating the nerves, and is the hallmark of some neurodegenerative, in particular autoimmune diseases. Contrary thereto, axonal loss is the destruction of the neuronal axon. There are important phenotypic, cellular, and molecular differences between demyelination and axonal loss. Similarly, the processes of myelin and axon regeneration, i.e. remyelination and axon regeneration, are characterised by distinct phenotypic, cellular and molecular processes. Axon regeneration primarily constitutes the regrowth of the axon from neuronal cells and reformation of new synapses, whereas remyelination is the process of developing a new myelin sheath on existing but demyelinated axons, which is mediated by differentiation of OPCs in the vicinity of the neural cells, or OPCs transplanted into the diseased CNS or cerebrospinal fluid (CSF) cavities, into new myelinating oligodendrocytes.

Axon regeneration has been e.g. described in the JP 2006/327942 (Abstract) wherein the use of Rho-kinase inhibitors, e.g. fasudil or Y-27632, has been proposed for the regeneration of axons. Sun et al. (Journal of Neuroimmunology 180 (2006): 126-134) mention the use of the Rho-kinase inhibitor fasudil for the treatment of experimental autoimmune encephalomyelitis. This treatment is explained by an anti-inflammatory effect of fasudil relating to the down regulation of IL-17. An anti-inflammatory therapy is the current method of choice in neurodegenerative autoimmune diseases such as multiple sclerosis, reducing the inflammatory attack on the CNS in particular by inhibiting or reducing the mobility and function of effector immune cells. Rho-kinase inhibitors (in particular fasudil) are also known as neuroprotective substances for induced stroke (Toshima et al., Stroke 2000; 31:2245-2250). The effect of neuroprotection is again explained by an anti-inflammatory effect resulting in a reduced severity or rate of the attack of immune effector cells on CNS tissue.

According to Sivasankaran et al. (Nature Neuroscience 7(3) (2004): 261-2268) axon regeneration is impaired by inhibitors of regenerative processes found in the myelin. These inhibitors have been identified as MAG, NOGO-A and OMgp. However, these inhibitors of axon regeneration do not impair regeneration of myelin sheaths as has been experimentally shown by Syed et al. (Neurosurg Focus 24 (2008): DOI: 10.3171/FOC/2008/24/3-4/E4). This further illustrates the significant differences between axon regeneration and remyelination. Although remyelination can also be inhibited by inhibitors found in myelin-debris resulting from demyelinating diseases, the relevant inhibitors differ from the inhibitors of axon regeneration due to differences in the mechanism.

At present, anti-inflammatory therapies are the method of choice for the treatment of neurodegenerative autoimmune diseases. However, the inhibition of immune effector cells has also adverse effects: The inhibition of the immune system also results in a reduction of the removal of myelin debris that contains inhibitors of regenerative processes (Franklin et al., Nature Reviews (3), (2002): 705-713; Franklin et al., J Neurol (2008) 255 [Suppl 1]: 19-25). Furthermore, it has been shown that anti-inflammatory treatment with e.g. corticoids results in an impairment in the restoration of myelin sheaths (Chary et al., Journal of Neuroscience Research 83 (2006): 594-605). Conversely, efficient removal of myelin-debris by macrophages may permit remyelination of damaged axons (Kotter et al., Glia 35 (2001): 204-212 ; Kotter et al., Neurobiol Dis 18 (2005): 6-175).

Although a number of treatment methods are available or are currently being evaluated, that aim at modulating the immune response to reduce the damage burden, or at neutralising inhibitors of axon regeneration to promote axon growth, there is still a lack of pharmaceutical agents that specifically promote the remyelination of axons. It is therefore the aim of the present invention to provide a method capable of restoring myelin sheaths on damaged or demyelinated axons of otherwise intact nerve cells.

Axonal integrity is tightly coupled with functional myelin, as subtle changes in the molecular composition of myelin can result in mid- to long term axonal degeneration under otherwise physiological conditions. The trophic function of myelin sheaths is also very likely to be of significance for maintaining axonal integrity in both acute and chronic CNS pathology, a situation in which denuded axons become more prone to injury or degeneration than axons bearing functionally intact myelin sheaths. Thus, the present invention also aims at methods and means by which remyelination is enhanced therapeutically and by which axons consequently are protected by formation of myelin sheaths.

### Summary of the invention

According to a first embodiment the present invention therefore relates to the use of an agent capable of enhancing the PKA-p38-CREB pathway, suppressing the Fyn-RhoA-Rock and/or PKC-alpha-MARCKS pathways or Ephrin-B3 or Sema3A for the manufacture of a pharmaceutical composition for the treatment of damaged myelin sheaths. The agent of the invention in particular neutralizes the effect of inhibitors of the regeneration of damaged myelin sheaths by oligodendrocytes. Inhibitors of remyelination include inhibitory myelin molecules, in particular Ephrin-B3 and inhibitory molecules of the extracellular matrix, in particular Sema3A. These inhibitors are in particular found in lesions in diseases associated with demyelination of axons. The inhibitors act on the PKA-p38-CREB, Fyn-RhoA-Rock and PKC-alpha-MARCKS pathways which are therefore also targets to promote remyelination.

Furthermore the invention provides an agent capable of enhancing the PKA-p38-CREB pathway, suppressing the Fyn-RhoA-Rock and/or PKC-alpha-MARCKS pathways or suppressing Ephrin-B3 or Sema3A for the treatment of damaged myelin sheaths, wherein the agent neutralizes the effect of inhibitors of the regeneration of damaged myelin sheaths by oligodendrocytes and the inhibitors include Ephrin-B3, Sema3A, which have been identified according to the invention, and myelin debris in general, which most likely contains further inhibitors. These inhibitors can be myelin molecules, such as Ephrin-B3, or molecules of the extracellular matrix, such as Sema3A.

According to the present invention three cellular pathways have been identified which can be modulated to promote myelin regeneration. This remyelination can in particular also be performed in the presence of inhibitors of myelin reformation found in myelin debris and or the glial scar. In addition, such inhibitors have been identified as being EphrinB3 and Sema3A which can be neutralized by e.g. treating oligodendrocyte precursor cells with agents modifying the three above mentioned signalling pathways, PKA-p38-CREB, Fyn-RhoA-Rock and PKC-alpha-MARCKS, or by using a sequestration agent, in particular antibodies directed at the inhibitors themselves in order to promote remyelination. All these approaches have in common that specifically remyelination is facilitated on neuronal axons after demyelination wherein the axons are, apart from the demyelination, structurally intact. This remyelination is facilitated by stem cells which have also been termed oligodendrocytes precursor cells, referred herein as OPCs, which are usually located in the vicinity of neuronal cells and axons. These stem cells have also been termed neuronal stem cells, tissue stem cells and adult stem cells. OPCs are thus stem cells capable of differentiation into active oligodendrocytes which perform a remyelination action. In a first stage of stem cell (OPC) differentiation pre-oligodentrocytes are formed. Pre-oligodendrocytes then develop into the active mature oligodentrocytes responsible for restoring myelin sheaths on axons, which is enhanced by the invention.

Failure of oligodendrocyte precursor cell (OPC) differentiation contributes significantly to failed myelin sheath regeneration (remyelination) in chronic demyelinating diseases. Although the reasons for this failure are not completely understood, the identified factors inhibit differentiation of cells capable of generating remyelinating oligodendrocytes and thus contribute to the failure of myelin regeneration. It was previously demonstrated that myelin debris generated by demyelination inhibits remyelination by inhibiting OPC differentiation and that the inhibitory effects are associated with previously unknown myelin proteins. Herein, it was shown that the inhibitory effects on OPC differentiation mediated by myelin are regulated by the Fyn-RhoA-ROCK, PKC-alpha-MARCKs and PKA-p38-CREB pathways, three cellular pathways of the stem cells which are modulated according to the present invention. It was demonstrated that pharmacological or siRNA-mediated inhibition of RhoA-ROCK-II and/or PKC signalling as well as enhancing PKA-p38-CREB signalling is able to induce OPC differentiation in the presence of myelin debris. The present results provide a mechanistic link between myelin, a mediator of OPC differentiation inhibition associated with demyelinating pathologies, and specific signalling pathways amenable to pharmacological manipulation, and therefore facilitate axon, inparticular CNS axon, remyelination. By assessing the activation of key molecules of the three pathways evidence of a direct involvement of Src family kinase Fyn and RhoA in mediating the inhibitory effects as well as a modulation of PKC-alpha signalling is provided.

The cascades that were found to be modulated by the presence of myelin inhibitors differ from the ones that regulate the inhibition of axon growth via NogoR and Lingo-1 signalling. There is no evidence that NogoR is expressed by OPCs and it was not possible to detect NogoR expression in cultures of OPCs at different time points. Furthermore, no evidence has been seen of an inhibitory effect of any of the classical myelin inhibitors of axon growth on OPC differentiation (Syed et al., Neurosurg Focus 24 (2008): E5). Thus, the molecular substrates in myelin mediating the inhibitory effects present new therapeutic targets. As has been shown in the examples, the dominant inhibitors of remyelination are Ephrin-B3 and Sema3A. The present results provide a model of how myelin exerts inhibitory effects on OPC differentiation. In inhibiting RhoA-ROCK and PKC-alpha signalling or enhancing PKA-p38-CREB signalling, pharmacological targets by which OPC differentiation can be powerfully stimulated in the context of myelin inhibitors have been identified.

The present invention thus provides novel methods to promote remyelination and treat demyelinated lesions, in particular in the presence of inhibitors of remyelination. The lesions can be acute or chronic. Both types of lesions contain inhibitors. EphrinB3, as identified by the present invention, as myelin associated inhibitor is found in acute lesions. Sub-acute and chronic lesions, in particular in a glial scar, comprise inhibitors such as Sema3A.

### The PKA-p38-CREB pathway

As has been found according to the present invention, the PKA-p38-CREB pathway promotes oligodendrocyte precursor cell differentiation into remyelinating oligodendrocytes. In preferred embodiments of the present invention the agent is an enhancing agent of the PKA-p38-CREB pathway, preferably selected from cAMP agonists or phosphodiesterase-4 inhibitors, PKA agonists, p38 MAPK agonists or CREB agonists, even more preferred being selected from rolipram (CAS 61413-54-5) or db-cAMP. The activity of CREB is particularly beneficial for the differentiation of oligodendrocyte precursor cells into active oligodendrocytes. This activity can be enhanced by either directly enhancing the activity of CREB or by enhancing one of the members of the PKA-p38-CREB pathway. At the beginning of this pathway cAMP acts activating on PKA, which in turn acts activating on p38 MAPK which in turn acts activating on CREB. Thus, the activation of anyone of cAMP, PKA, p38 MAPK, CREB promotes differentiation. Activators are e.g. the proteins themselves, nucleic acids encoding these proteins, or inhibitors of enzymes which deactivate members of the pathway. Such an enzyme is e.g. phosphodiesterase-4 (PDE-4) which degrades cAMP. A PDE-4 inhibitor which inhibits degradation of cAMP can thus be used. Inhibitors are e.g. small molecule inhibitors or agents of RNAi (RNA interference), in particular siRNAs (small interfering RNAs). However, the term "small" should not be construed as the RNAi agents being small RNA molecules, since the small interfering RNA agents are actually produced in the cell itself and as pharmaceutical agent also larger oligonucleotide sequences e.g. with more than 12, more than 15, more than 20, more than 30, more than 40, more than 50, more than 60, more than 70, more than 80, more than 90, more than 100, more than 150, more than 180, more than 200, more than 250, more than 300, more than 400, or even more than 500 nucleotides (or base pairs) can be used with a suitable carrier facilitating entry of the RNAi agent into cells. Such carriers are e.g. liposomes facilitating oligonucleotide entry into a cell. The RNAi agent is preferably an oligonucleotide sequence of up to 1000, up to 600, up to 500, up to 400, up to 300, or up to 200 nucleotides (or base pairs). An example of an enhancing agent of cAMP furthermore is db-cAMP (CAS 16980-89-5). In further preferred embodiments an enhancer of this pathway is rolipram, which is a small molecule phosphodiesterase-4 inhibitor. Other phosphodiesterase-4 inhibitor are theophylline and AWD 12-281 (N-(3,5-dichloropyrid-4-yl)-[1-(4-fluorobenzyl)-5-hydroxy-indole-3-yl]-glyoxylic acid amide). Further preferred small molecule agents enhancing the PKA-p38-cAMP pathway are Milrinone (CAS 78415-72-2), Zl-n-91, Pyrazolopyridines as disclosed in the US 4,925,849 B, Cl-1044 (NLM ID C418118, N-(9-amino-4-oxo-1-phenyl-3,4,6,7-tetrahydro(1,4)diazepino(6,7,1-hi)i=dol-3-yl)nicotinamide), Tetomilast (CAS 145739-56-6), oglemilast (CAS 778576-62-8), apremilast (CAS 608141-41-9), ONO 6126, having the following chemical formula: 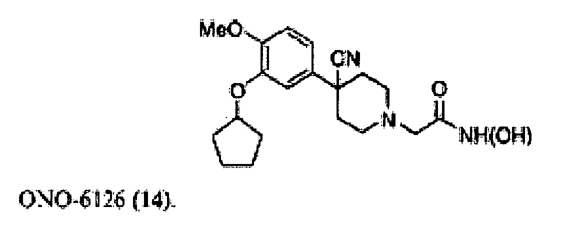 IPL-512602 (a synthetic analogue of the steroid contignasterol (CAS 137571-30-3) isolated from the sponge Petrosia contignata), IPL-455903 (HT-0712, CAS 617720-02-2, (3S, 5S)-5-(3-cyclo-pentyloxy-4-methoxy-phenyl)-3-(3-methyl-benzyl)-piperidin-2-one)), Roflumilast (CAS 162401-32-3), Cilomilast (CAS 0031431-43-3, 4-cyano-4-(3-cyclopentyloxy-4-methoxyphenyl)cyclohexane-1-carboxylic acid), GRC 3886, CC-11050 (Celgene), Mesembrine (CAS 468-53-1), Ibudilast (CAS 50847-11-5), Mesopram (Daxalipram, PubChem 5287536, CAS 189940-24-7), Tofimilast (NLM ID C517806, CAS-No. 185954-27-2, 9-cyclopentyl-5,6-dihydro-7-ethyl-3-(thien-2-yl)-9H-pyrazolo(3,4-c)-1,2,4-triazolo(4,3-a)pyridine)

### The Fyn-RhoA-Rock pathway

In further preferred embodiments the agent is a suppressing agent of the Fyn-RhoA-Rock pathway, in particular selected from ROCK inhibitors, Fyn agonists, preferably being selected from RNAi of RhoA, RNAi of ROCK-II, or combinations thereof. Also preferred is an agent being a suppressing agent of the Fyn-RhoA-Rock pathway, preferably being a Rho inhibitor, more preferred being Fasudil or Y-27632, for the combined administration with an anti-inflammatory agent.

In the Fyn-RhoA-Rock pathway, Rock is an inhibitor of oligodendrocyte precursor cell differentiation into active oligodendrocytes (or into remyelinating oligodendrocytes) capable of remyelination. Thus, inhibition of Rock negates this inhibitory action on the differentiation, which in turn promotes differentiation. In this pathway RhoA is activated into RhoA-P. This reaction is suppressed by Fyn. RhoA-P in turn activates Rock, the primary inhibitor of differentiation, and thus is a target for a suppression therapy in order to promote remyelination. Preferred inhibitors modulating the Fyn-RhoA-Rock pathway are thus Rock inhibitors, Fyn agonists, and RhoA inhibitors. Inhibitors are e.g. small molecule inhibitors or agents of RNAi (RNA interference), in particular siRNAs (small interfering RNAs). However, the term "small" should not be construed as the RNAi agents being small RNA molecules since the small interfering RNA agents are actually produced in the cell itself and as pharmaceutical agent also larger oligonucleotide sequences e.g. with more than 12, more than 15, more than 20, more than 30, more than 40, more than 50, more than 60, more than 70, more than 80, more than 90, more than 100, more than 150, more than 180, more than 200, more than 250, more than 300, more than 400, or even more than 500 nucleotides (or base pairs) can be used with a suitable carrier facilitating entry of the RNAi agent into cells. Such carriers are e.g. liposomes. The RNAi agent is preferably a oligonucleotide sequence of up to 1000, up to 600, up to 500, up to 400, up to 300, or up to 200 nucleotides (or base pairs). Preferred suppressing agents of the Fyn-RhoA-Rock pathway are thus RNAi agents of RhoA, of Rock (in general or as individual RockI or RockII inhibitors), in particular of Rock-II. Further preferred suppressing agents are HA-1077 (fasudil; CAS103745-39-7) and Y-27632 (CAS 331752-47-7) which are both RhoA inhibitors. Further suppressing agents are isoquinolin derivatives acting on Rho, in particular the Isoquinoline 6-piperidinyl-substituted isoquinoline derivatives disclosed in the W02007/000240 A1, Hydroxyfasudil (CAS 155558-32-0, VWR 390602-2) acting on Rock, Y-39983 (National Library of Medicine, Substance ID C520365; BIO Clinica, 17 [13], 1191-1194 [2002]), Y-30141. Further agents are BA210 (Cethrin, engineered protein variant of C3, mage by BioAx-one), or Y-27623, Y-30141, Y-33075, Y-32885, Y-30964, Y-28791, and H-7 disclosed in US6,218,410 B1 and W02003/105923.

Fasudil and Y-27632 have been previously mentioned as agents capable of promoting axon regeneration. As has been explained in the introduction, there is a significant difference between the inventive treatment of damaged myelin sheaths and axon regeneration (reformation of complete axons from neuronal cells). The effect on remyelination has not been mentioned previously for fasudil and Y-27632. Fasudil and Y-27632 and other RhoA inhibitors are nevertheless preferably used in combination with an anti-inflammatory agent or in combination with an oligodendrocyte precursor cell or pre-oligodendrocyte stem cell therapy, explained below. In particular the agent is for the combined therapy with a pre-oligodendrocyte or oligodendrocyte precursor cell or embryonic or adult or olfactory bulb derived stem cell therapy. This combined use benefits from a synergistic effect. According to the present invention it has been found that use of a RhoA inhibitor, as any of the inventive enhancing or suppressing agents, facilitates differentiation of oligodendrocyte precursor cells into active oligodendrocytes, the cellular factors of myelin regeneration, even in the presence of differentiation inhibitors. Such differentiation inhibitors are usually found in myelin debris or the glial scar formed in demyelinating lesions. Such inhibitors are e.g. Ephrin-B3 and Sema3A. In demyelinating diseases the present standard therapy is the administration of anti-inflammatory agents which should prevent further demyelination. However, such anti-inflammatory agents also suppress the removal of myelin debris since action and mobility of immune cells is impaired (Franklin et al., J Neurol (2008) 255 [Suppl 1]: 19-25) and impair remyelination (Chary et al, Journal of Neuroscience Research 83 (2006): 594-605). The combined use of an anti-inflammatory agent with the inventive Fyn-RhoA-Rock suppressing agent, in particular a RhoA inhibitor such as Fasudil or Y-27632, enhances the therapy since remyelination is now possible in the presence of myelin debris.

### The PKC-alpha-MARCKS pathway

In further preferred embodiments the agent is a suppressing agent of the PKC-alpha-MARCKS pathway, preferably a PKC-alpha inhibitor, more preferred being selected from RNAi of PKC-alpha, Gö-6976 (CAS 136194-77-9) or BIM (Ro 31-8220; CAS 125314-64-9). In the PKC-alpha-MARCKS pathway PKC-alpha acts as an inhibitor of the differentiation of oligodendrocyte precursor cells into active oligodendrocytes capable of remyelination, and thus the inhibition of PKC-alpha removes its inhibition on the differentiation. Therefore, preferred suppressing agents according to the invention are PKC-alpha inhibitors such as RNAi (RNA interference) agents of PKC-alpha. Preferably the RNAi agents are oligonucleotide sequences with more than 12, more than 15, more than 20, more than 30, more than 40, more than 50, more than 60, more than 70, more than 80, more than 90, more than 100, more than 150, more than 180, more than 200, more than 250, more than 300, more than 400, or even more than 500 nucleotides (or base pairs). These agents can be used with a suitable carrier, e.g. liposomes, facilitating the entry of the RNAi agent into the cells. Further preferred RNAi agents have an oligonucleotide sequence of up to 1000, up to 600, up to 500, up to 400, up to 300, or up to 200 nucleotides (or base pairs). Preferred PKC-alpha inhibitors are small molecule inhibitors such as Gö-6976 (CAS 136194779) and BIM (CAS 125314649). Further suppressing agent according to the invention are staurosporin (CAS 62996-74-1), CGP41251 (CAS 120685-11-2), Bryostatin-1 (CAS 83314-01-6), Aprinocarsen (CAS 331257-53-5), Midostaurin (PKC412, 4'-N-benzoyl staurosporine), AffinitakTM(ISIS-3521), AEB071 (NVP-AEB071), UCN01 (CAS 112953-11-4, 7-Hydroxystaurosporine), ISIS 9606 (a 20-mer phosphorothioate oligonucleotide targeting the 3-untranslated region of the human PKC-mRNA, which contains 2-methoxyethyl modifications incorporated into the 5 and 3 segments of the oligonucleotide:

| OLIGO# | STRUCTURE AND SEQUENCE | T_{M} | SUBSTITUENT |
|---|---|---|---|
| ISIS 3521 | GsTsTsCsTsCsGsCsTsGsGsTsGsAsGsTsTsTsCsA | 52.1 | 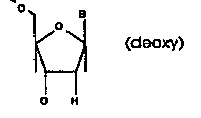 |
| ISIS 11485 | GoToToCoToCoGoCoToGoGoToGoAoGoToToToCoA | ND | |
| ISIS 5357 | GsTsTsCsTsCsGsCsTsGsGsTsGsAsGsTsTsTsCsA | 61.9 | 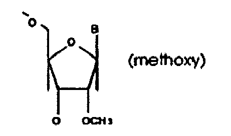 |
| ISIS 8329 | GoToToCoToCoGsCsTsGsGsTsGsAsGoToToToCoA | 62.6 | |
| ISIS 9606 | GsTsTsCsTsCsGsCsTsGsGsTsGsAsGsTsTsTsCsA | 64.8 | 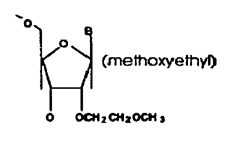 |
| ISIS 9605 | GoToToCoToCoGsCsTsGsGsTsGsAsGoToToToCoA | 69.6 | |

, Antisense nucleotides, e.g. directed at the 5'- or 3'-untranslated region, or the coding region of PKC-alpha, such as of the following sequences:

| Oligonucleotide No. | Sequence (5' - 3') | cDNA target | PKC-α |
|---|---|---|---|
| | | | % control |
| 3520 | CCCCAACCACCTCTTGCTCC | 5'-Untranslated | 39 ± 5 |
| 3522 | AAAACGTCAGCCATGGTCCC | AUG codon | 39 ± 4 |
| 3674 | CCCGGGAAAACGTCAGCCAT | AUG codon | 52 ± 4 |
| 3890 | GTCAGCCATGGTCCCCCCCC | AUG codon | 46 ± 4 |
| 3891 | CGCCGTGGAGTCGTTGCCCG | Coding (V1) | 54 ± 8 |
| 3682 | CTGCCTCAGCGCCCCTTTGC | Coding (C1) | 47 ± 4 |
| 3686 | AGTCGGTGCAGTGGCTGGAG | Coding (C1) | 88 ± 7 |
| 3687 | GCAGAGGCTGGGGACATTGA | Coding (C1) | 81 ± 4 |
| 3886 | ATGGGGTGCACAAACTGGGG | Coding (C3) | 94 ± 10 |
| 3892 | TCAAATGGAGGCTGCCCGGC | Coding (C3) | 66 ± 7 |
| 3521 | GTTCTCGCTGGTGAGTTTCA | 3'-Untranslated | 52 ± 2 |
| 3526 | GGATTCACTTCCACTGCGGG | 3'-Untranslated | 82 ± 3 |
| 3527 | GAGACCCTGAACAGTTGATC | 3'-Untranslated | 61 ± 4 |
| 3695 | GGGCTGGGGAGGTGTTTGTT | 3'-Untranslated | 95 ± 6 |
| 3875 | CACTGCGGGGAGGGCTGCGG | 3'-Untranslated | 101 ± 5 |
| 3878 | AGCCGTGGCCTTAAAATTTT | 3'-Untranslated | 84 ± 8 |
| 3879 | ATTTTCAGGCCTCCATATGG | 3'-Untranslated | 82 ± 5 |
| 3884 | AAGAGAGAGACCCTGAACAG | 3'-Untranslated | 62 ± 4 |
| 3885 | GATAATGTTCTTGGTTGTAA | 3'-Untranslated | 85 ± 7 |
| 3947 | TGGAATCACACACAAGCCGT | 3'-Untranslated | 89 ± 8 |

, Tamoxifen (CAS 10540-29-1), Bryostatin 1 (CAS 83314-01-6), Calphostin C (CAS 121263-19-2), GF109203X (CAS 133052-90-1), Ro 31-7549 (CAS 125313-65-7), Gö 6983 (CAS 133053-19-7, 2-[1-(3-di-methylaminopropyl)-5-methoxyindol-3-yl]-3-(1H-indol-3-yl) maleimide), Chelerythrine chloride (CAS 3895-92-9), LY-333351 (CAS 169939-94-0) or (-)-Balanol:

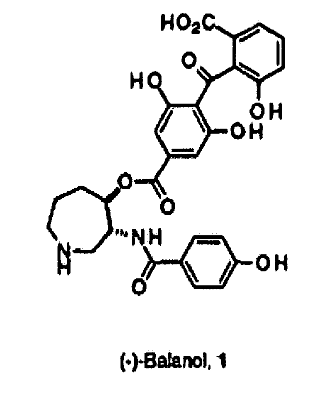

### Remyelination Inhibitors

According to the present invention Ephrin-B3 and Sema3A are inhibitors of remyelination present in myelin debris and / or the glial scar. Myelin debris in demyelinating diseases forms near axons and constitutes a potent inhibitor of remyelination by inhibiting oligodendrocyte precursor cell differentiation and activation. According to the invention it was found that Ephrin-B3 and Sema3A prevent the differentiation of oligodendrocyte precursor cells into active oligodendrocytes, which in turn perform the remyelinating actions. By removing one or more of these inhibitors the remyelination efforts can be increased. Therefore, the present invention provides in a further aspect, the use of an agent capable of suppressing Ephrin-B3 and/or Sema3A for the treatment of damaged myelin sheaths. Preferably the agent can suppress the inhibitory action of Ephrin-B3, e.g. by removing Ephrin-B3 or sequestering, i.e. masking, its effect or function on oligodendrocyte precursor cells. Furthermore, the agent can suppress the inhibitory action of Sema3A, e.g. by removing Sema3A or sequestering, i.e. masking, its effect or function on oligodendrocyte precursor cells. Such agents that can suppress the actions of Ephrin-B3 or Sema3A are in particular binding agents. Agents that bind inhibitory components of myelin debris and / or the glial scar are e.g. natural ligands or antibodies. Such antibodies can be polyclonal or monoclonal antibodies. Preferably the antibodies are polyclonal or a mixture of different monoclonal antibodies. In particular a combination of antibodies recognizing 2, preferably 3, more preferred 4, even more preferred 5, also preferred 6, or more different epitopes on EphrinB3, but also on Sema3A, are particularly efficient. Using differnt antibodies enhances the masking effect of these inhibitors. These agents can be used as pharmaceutical components for the preparation of a medicament for the treatment of demyelinating diseases and/or to promote the repair of damaged myelin sheaths.

The agent of the present invention preferably neutralizes, e.g. sufficiently sequesters, masks or removes, the inhibitors of oligodendrocyte precursors cell differentiation to active oligodendrocytes. The sufficient amount of such an agent capable of the neutralization can e.g. be predetermined in a batch assay or continuously administered in increasing doses to a patient who is monitored for the inhibitory actions of e.g. Ephrin-B3 or Sema3A or further inhibitors of the differentiation found in myelin debris.

### Therapeutic uses and targets

The present invention provides a specific method to treat conditions in which myelin sheaths are damaged. This myelin sheath damage is in preferred embodiments associated with axon demyelination. In particular this damage is on structurally undamaged axons (apart from the demyelination around the axons). Thus, a preferred subclass of diseases is characterized by axons which are not destroyed. The damage on the myelin sheath can e.g. be induced by inflammation, in particular in the case multiple sclerosis. Further demyelinating diseases are in general neurodegenerative diseases, including multiple sclerosis, acute disseminated encephalomyelitis, transverse myelitis, Alexander's disease, Canavan disease, Cockayne's syndrome, Pelizaeus-Merzbacher's disease, optic neuritis, hereditary leukencephalopathy, Guillain-Barre Syndrome and central pontine myelinosis, deep white matter ischemia, progressive multifocal leukoencephalopathy, post-infectious encephalitis, demyelinating HIV Encephalitis, demyelinating Radiation Injury, Acquired toxic-metabolic disorders, posterior reversible encephalopathy Syndrome, central pontine myelinolysis, hereditary metabolic disorders, including leukodystrophies, in particular Adrenoleukodystroph, Krabbe's globoid cell, metachromatic leukodystrophy. In particular the treatment is directed at the demyelinating aspects or subclasses of these diseases.

Deep white matter ischemia is a typical age-related disease. As the brain ages, structural, chemical, and metabolic changes occur that are reflected on the MR images. Most important for MR interpretation are the changes related to deep white matter ischemia. The deep white matter of the cerebral hemispheres receives its blood supply from long, small-calibre arteries and arterioles that penetrate the cerebral cortex and traverse the superficial white matter fiber tracts. The white matter does not have as generous a blood supply as the gray matter and is more susceptible to ischemia. As the nutrient arteries become narrowed by arteriosclerosis and lipohyaline deposits within the vessel walls, the white matter becomes ischemic on a chronic basis.

Multiple sclerosis (MS), to be treated according to the present invention by remyelination, is characterized by loss of myelin with sparing of axon cylinders in MS plaques. The plaques usually occur in a perivenular distribution and are associated with a neuroglial reaction and infiltration of mononuclear cells and lymphocytes. Active demyelination is accompanied by transient breakdown of the blood-brain barrier. Chronic lesions show predominantly gliosis in the form of glial scar tissue. MS plaques are distributed throughout the white matter of the optic nerves, chiasm and tracts, the cerebrum, the brain stem, the cerebellum, and the spinal cord.

Progressive multifocal leukoencephalopathy has an affinity for subcortical white matter, and the classic distribution is in the parietal-occipital lobes. It is not primarily a periventricular process, but as the disease progresses, the deeper white matter is also affected. Any white matter structures can be involved, but lesions of the corpus callosum are much less common than in multiple sclerosis, for example.

Post-infectious encephalitis, also called acute disseminated encephalomyelitis (ADEM), is a demyelinating disease that is thought to be of autoimmune origin. It usually occurs within two weeks after one of the childhood viral infections, such as measles or chicken pox, or following vaccination against rabies or smallpox. It has also been reported in association with chronic Epstein-Barr virus infection. The clinical picture is one of abrupt onset with a monophasic course, to distinguish it from MS and most other white matter diseases. Initial headache, seizures, and drowsiness may progress to profound lethargy and even coma. Brain stem involvement can produce nystagmus, diplopia, and dysarthria. Since it is a myelinoclastic process, lesions usually correlate with discrete clinical symptoms.

HIV Encephalitis: A subacute encephalitis involving the white matter is seen in up to 30% of patients with AIDS related to direct invasion of neurons by the neurotropic retrovirus, human immunodeficiency virus type 1 (HIV). Presenting symptoms include headache, memory loss, language difficulty, movement disorders, and various sensory deficits. More advanced disease is signaled by behavioral disorders, loss of bowel and bladder control, and the AIDS dementia complex. HIV and cytomegalovirus often coexist in brain specimens taken from patients with AIDS; however, HIV seems to be the predominant etiology for the microglial nodules and multinucleate giant cells, the pathologic hallmarks of the white matter encephalitis.

Demyelinating radiation injury to the brain has a progressive and insidious course. The effects on the brain can be focal or diffuse, depending on whether whole brain or more focused radiation was given. A few months following radiation, demyelination is seen histologically, associated with proliferation of the glial elements and mononuclear cells.

Acquired toxic-metabolic disorders relate to effects of chemotherapeutic agents. Many of the cancer chemotherapeutic drugs are neurotoxic. The blood-brain barrier (BBB) limits the direct entry of these substances into the brain, but the BBB may be damaged by the disease process or by the therapeutic agent itself. Also, in the case of CNS disease, agents may be given to open the BBB to allow greater access of the anticancer drug to the tumor. Lipid solubility and intrathecal administration are other factors that increase delivery to the brain. The chemotherapeutic agents commonly associated with leukoencephalopathy include methotrexate, cis-platin, cytosine arabinoside (ARA-C), carmustine (BCNU), and thiotepa.

The posterior reversible encephalopathy syndrome (PRES) describes the combination of posterior cerebral involvement and brain dysfunction that occurs with this entity. Patients may present with headache, confusion, visual disturbance, and seizures. MRI reveals lesions with high signal intensity on FLAIR and T2 weighted non-enhanced images. There is a predilection for the posterior cerebral white matter, and in severe cases the adjacent gray matter can be involved. Extension into the frontal lobes is not uncommon, but isolated anterior white matter involvement is unusual.

Central pontine myelinolysis, also called osmotic demyelination syndrome, is a disorder characterized pathologically by dissolution of the myelin sheaths of fibers within the central aspect of the basis pontis. In extreme cases there may be extension to the pontine tegmentum, midbrain, thalamus, internal capsule, and cerebral cortex. The myelinolysis occurs with relative sparing of the nerve cells and axon cylinders.

Another class of diseases to be treated according to the present invention, in particular their demyelination aspect, are heredetary metabolic diseases with demyelination. Included in this group are the classic leukodystrophies including adrenoleukodystrophy, Krabbe's globoid cell, and metachromatic leukodystrophy, and a few other less well known entities. They have in common a genetic origin and involve the peripheral nerves as well as the central nervous system. Each is caused by a specific inherited biochemical defect in the metabolism of myelin proteolipids that results in abnormal accumulation of a metabolite in brain tissue. Progressive visual failure, mental deterioration, and spastic paralysis develop early in life, however, variants of these diseases have a more delayed onset and a less progressive course. The other primary white matter disorders include Alexander's disease, Canavan disease, Cockayne's syndrome, and Pelizaeus-Merzbacher's disease. All of the above white matter diseases are characterized by symmetric massive involvement of the white matter. MR imaging is very sensitive for detecting the white matter damage. Two further metabolic disorders, Hurler's disease and Lowe's syndrome, are associated with cystic changes in the cerebral white matter.

A further demyelinating disease or condition that can be treated according to the present invention is heavy metal poisoning, in particular mercury poisoning. With the inventive treatment (reversible) axon demyelination can be reversed by remyelination. Contrary thereto, axon destruction is irreversible and requires a complete regrowth of a new axon which in turn has to form new neuronal connections and synapses. According to the treatment by the agents of the invention synapses and neural connections are maintained. The inventive treatment is directed specificall at myelin damage, i.e. the treatment of myelin damage but not of axon destruction.

Remyelination usually involves the recruitment phase and demyelination phase. Preferably, in a disease or condition with damaged myelin sheaths to be treated according to the invention, OPCs are normally recruited, i.e. there is no recruitment failure and oligodendrocyte precursor cells are located around the axons. According to the invention a further step of remyelination is specifically addressed, i.e. overcoming remyelination failure in the differentiation phase.

The cellular target of the present invention are oligodendrocyte precursor cells (OPCs) (i.e. adult, neural, tissue stem cells) and pre-oligodendrocytes. But also in general any neuronal precursor cells that can be differentiated into active oligodendrocytes is targeted. The first step in remyelination is the population of an area of demyelination with sufficient OPCs. These might come from cells that are already present in the demyelinated area, or they may be recruited from surrounding intact white matter or may be artificially brought to the affected area by transplantation. This initial recruitment phase may involve OPC proliferation and is also likely to involve their migration. The contribution made by OPC migration to remyelination is likely to be determined by the extent to which OPCs survive in areas of demyelination. Once recruited, the OPCs must differentiate into myelin-sheath-forming oligodendrocytes for remyelination to be completed. During this differentiation phase, the OPCs differentiate into pre-myelinating oligodendrocytes that engage the demyelinated axon, before finally becoming mature oligodendrocytes as their sheet-like processes form spiral wraps around axons and the cytoplasmic contents of the wraps are extruded to form compacted myelin sheaths (Franklin, Nature Reviews Neuroscience 3 (2002): 705-714).

Demyelinated axons to be treated according to the present invention are preferably found or associated with diseases or conditions selected from multiple sclerosis, stroke, nerve injury, in particular spinal cord injury. Nerve injury may be of central nervous system axons, of corticospinal tract axons, of dorsal column axons, or of optic nerve axons. Furthermore, the denuded axons to be treated according to the present intervention include known subtypes of multiple sclerosis, in particular relapse-remitting MS, secondary progressive MS, and primary progressive MS as well as fulminant forms of MS. Furthermore, MS types to be treated include MS classified as Type 1, as MS Type 2, as MS Type 3, and as MS Type 4 according to their pathogenesis (Lassmann et al., TRENDS in Molecular Medicine 7;3 (2001): 115-121).

The damaged myelin sheaths can be of central nerve system (CNS) axons, in particular of corticospinal tract axons, of dorsal column axons, optic nerve axons, optic tract axons or of axons in the penumbra after a stroke or after spinal cord injury. Another characteristic of the damage of the myelin sheath according to the invention can be a chronic damage, or a damage as a result of a chronic disease. Preferably in the chronic damage or chronic disease a glial scar is formed, in particular a glial scar comprising Sema3A. In further preferred embodiments the damage of the myelin sheath can be an acute or sub-acute damage, preferably resulting in the presence of myelin associated inhibitors, in particular Ephrin B3. In particular the myelin sheath damage is treated after an acute surge during the chronic disease or is treated continuously.

The importance of myelin sheaths and their restoration to spinal cord injury (SCI) oligodendrocyte death is a prominent feature of SCI. Furthermore, following SCI chronic progressive demyelination may occur. Whereas preventing oligodendrocyte cell death has beneficial effects on the recovery following spinal cord injury, the transplantation of OPCs improves white matter sparing as well as it promotes functional recovery. In the light of these findings, enhancing remyelination has been identified as important strategy to ease the devastating clinical manifestations of SCI.

In preferred embodiments of the present invention the agent is for the administration after complete demyelination of at least a portion of the neuronal axons of a subject, which can e.g. be in respects undamaged. This portion of neuronal axons is preferably a portion of central nerve system axons, of corticospinal tract axons, of dorsal column axons, optic nerve axons, optic tract axons or of axons in the penumbra after a stroke or after spinal cord injury, and especially preferred an optic nerve axon.

The subject for the inventive treatment is preferably a mammal, in particular a human.

### Therapeutic administration

The agent of the present invention can be administered after e.g. one hour, two hours, three hours, four hours, five hours, six hours, seven hours, eight hours, ten hours, twelve hours, eighteen hours or after twenty-four hours, i.e. after one day, preferably after three days, more preferably after one week, even more preferably after two weeks or after three weeks, after four weeks, after one month, after two month or even after three month after the onset of axon demyelination. In preferred embodiments the agent is for administration after one day, preferably after three days, more preferably after one week, even more preferably after two weeks or after three weeks, after four weeks, after one month, after two month or even after three month of the complete demyelination of at least a portion of the axons of the subject. Particular preferred a Rho inhibitor is administered at these time scales, but also administration of other agents according to the invention, in particular rolipram, is possible. This portion can e.g. be up to 0.1%, up to 0.5%, up to 1%, preferably 2%, more preferred 5% or even up to 10% or at least 0.00001%, at least 0.0001%, at least 0.001%, at least 0.01%, at least 0.1%, at least 1%, at least 2% or even at least 5% of the axons, which are usually myelinated in a healthy subject. Clinical symptoms might arise with only 0.0001% affected axons or e.g. 0.1% depending on the damaged/demyelinated areas. These axons are preferably central nerve system axons, of cord corticospinal tract axons, dorsal column axons, optic nerve axons, optic tract axons or axons in the penumbra after a stroke or after spinal cord injury. Preferably, the demyelinated axons are optic nerve axons, which are readily accessible and identified.

The administered dose of the present agents can be administered in usual pharmaceutically acceptable doses at common intervals for these compounds. Preferably, the agent is Rolipram which can e.g. be administered in a therapeutic dose of preferably 0.01 mg/kg per day to 10 mg/kg per day, more preferred 0.05 mg/kg per day to 2 mg/kg per day, most preferred 0.1 mg/kg per day to 0.8 mg/kg per day. In preferred embodiments a suppression of one of these pathways or differentiation inhibiting target (Ephrin-B3 or Sema3A) is an inactivation of the pathways or the target, preferably an inactivation of Ephrin-B3.

Preferably the enhancement of one of the enzymatic targets of the pathways includes the suppression of an endogenous suppressor of the pathways (e.g. in the case of enhancing the PKA-p38-CREB pathway) and cAMP turnover by phosphodiesterase-4 (PDE4), inhibited by PDE-4 inhibitors, acting as cAMP agonists or enhancers.

### Combinatory therapies

As has been shown in the examples enhancing PKA-p38-CREB, or suppressing the Fyn-RhoA-Rock or suppressing the PKC-alpha-MARCKS pathway or suppressing Ephrin-B3 or even Sema3A can lead to sufficient remyelination. However, better effects i.e. synergistic effects have been shown by a combination of at least two of these pathways or by combining modulation of one, two, three of these pathways and suppressing Ephrin-B3 and/or Sema3A, in particular suppressing Ephrin-B3. A particular preferred embodiment is the use of agent inhibiting the PKC-alpha-MARCKS pathway and inhibiting the Fyn-RhoA-Rock pathway. In general the present invention relates in a further prefer embodiment to the use of more than one agents selected from agents capable of enhancing the PKA-p38-CREB pathway or of suppressing the Fyn-RhoA-Rock and/or PKC-alpha-MARCKS pathways or suppressing Ephrin-B3 or Sema3A for administration together with at least one, preferably at least two additional agents suppressing a different pathway or molecular target of the pathways or target selected from Ephrin-B3 or Sema3A. Preferably the agent of the present invention is for combined use to enhance PKA-p38-CREB and suppress Fyn-RhoA-Rock, suppress Fyn-RhoA-Rock and PKC-alpha-MARCKS enhance PKA-p38-CREB and suppress PKC-alpha-MARCKS pathways.

In a further preferred embodiment the agent is for combined administration with an anti-inflammatory agent, preferably a corticosteroid, cortisone, mitoxantron, natalizumab, interferon beta or glatiramer acetate. Although inflammation is considered to play a causal role in the genesis of many demyelinating lesions, there is evidence that inflammation is also beneficial for repair. Remyelination is often associated with a prominent inflammatory response in experimental and MS lesions with studies demonstrating an impairment of remyelination in the absence of lymphocytes, MHC Class II antigens, inflammatory cytokines, macrophages, and inhibition of microglial activation. Corticosteroids are likely to exert many effects within the context of CNS inflammatory disease. Corticosteroids can: 1) induce apoptosis of blood leukocytes especially CD4+ T-cells, thereby reducing or terminating CNS inflammation, 2) act at a number of levels of the proinflammatory signalling cascades and reduce cellular infiltration and 3) act on memory and defence functions of macrophages and modify production of inflammatory cytokines, possibly by altering gene transcription. Given the evidence that inflammation plays an important role in promoting remyelination, such anti-inflammatory effects of CS can have a detrimental effect on repair mechanisms (Chary et al, Journal of Neuroscience Research 83 (2006): 594-605). The anti-inflammatory molecules are considered to be different molecules to the agents which promote remyelination of the invention used as additional pharmaceutical. In further embodiments, in particular in case of using two agents, preferably one agent to be used according to the invention has also an inflammatory effect.

Macrophages are mediators of CNS demyelination but are also important for remyelination because of their action as myelin debris removers. If clearance of myelin debris is disturbed by alterations of the inflammatory response, the presence of myelin debris can persist and thus for a prolonged period inhibit myelin repair. Recent studies suggest that remyelination requires the signalling environment provided by an acute inflammatory response (Kotter et al. 2005, supra). According to the present invention it is now possible to sufficiently provide an anti-inflammatory agent, in particular usual pharmaceuticals used to treat neurodegenerative autoimmune diseases such as multiple sclerosis, acute disseminated encephalomyelitis, transverse myelitis, Alexander's disease and central pontine myelinosis. The adverse effect on myelin debris removal is compensated by the use of the inventive agent, preferably in combination with more than one inventive agent which facilitates the remyelination despite the presence of remyelination inhibitors found in myelin debris. Preferred inventive agents used for a combination with an anti-inflammatory agent are RNAi agents of RhoA, Rock-II, Isoquinoline derivatives, Hydroxyfasudil, Y-39983, Y-30141, db-cAMP, Phosphodiesterase-4 inhibitors (in particular rolipram), Milrinone, Zl-n-91, Pyrazolopyridines, Cl-1044, Tetomilast, oglemilast, apremilast, ONO 6126, IPL-512602, IPL-455903 (aka HT-0712), Roflumilast, Cilomilast, GRC 3886, RNA agents of PKC-alpha, Gö-6976 (CAS 136194-77-9), BIM (Ro 31-8220; CAS 125314-64-9), Staurosporin, CGP41251, Bryostatin-1, Aprinocarsen, Midostaurin (PKC412), Affinitak^{™} (ISIS-3521), AEB071, UCN01, ISIS 9606, (Tamoxifen), and Antisense nucleotides. Further inventive agents to be used in combination with an anti-inflammatory agent are compounds that sequester, mask, bind or inactivate Ephrin-B3, or Sema3A. In particular preferred is the combination of the anti-inflammatory agents with anti-Ephrin-B3 antibodies or anti-Sema3A antibodies.

In a further preferred embodiment of the invention the inventive agents, enhancers of the PKA-p38-CREB pathway, suppressors of the Fyn-RhoA-Rock pathway, suppressors of the PKC-alpha-MARCKS pathway, suppressors of Ephrin-B3 or suppressors of Sema3A, in particular antagonists, sequestering agents, masking agents of Ephrin-B3, or RhoA-inhibitors, most preferred Rolipram, are for combined treatment with a stem cell therapy. According to this therapy the agents are together provided in the course of a therapy aimed at the administration of oligodendrocyte precursor cells or pre-oligodendrocytes to a subject. These cells can e.g. be administered systemically or locally, in particular at the site of demyelination or into the CSF compartments of the brain or intrathecally. The administration of these cells leads to further multiplication of the cells which can differentiate to the active myelin forming oligodendrocytes by circumventing differentiation inhibitors according to the invention.

### Kits

In a further aspect of the present invention a kit is provided comprising at least two, preferably three, four, five or more pharmaceutical compounds selected from at least two groups of the following groups:
group 1: cAMP agonists or phosphodiesterase-4 inhibitors, PKA agonists, p38 MAPK agonists or CREB agonists;
group 2: ROCK inhibitors, Fyn agonists and Rho inhibitors;
group 3: PKC-alpha inhibitors,
group 4: Ephrin-B3 inhibitors or Sema3A inhibitors. Preferably the agents include agents from at lest three or four groups.

Such a kit can be provided for the combined administration of the inventive agents, in particular further combined with an anti-inflammatory agent. Therefore, in a preferred embodiment a kit is provided comprising at least one pharmaceutical compound selected from the following groups:
group 1: cAMP agonists or phosphodiesterase-4 inhibitors, PKA agonists, p38 MAPK agonists or CREB agonists;
group 2: ROCK inhibitors, Fyn agonists and Rho inhibitors;
group 3: PKC-alpha inhibitors,
group 4: Ephrin-B3 inhibitors or Sema3A inhibitors; and an anti-inflammatory agent, preferably for the combined administration for the treatment of myelin sheath damage.

In the Kit the inventive agent acting on one or more of the above mentioned pathways can be provided separately, e.g. in separate solutions, emulsions or dispersions, or as solid compound, such as powders, tablets or capsules.

Preferred kits comprising a combination of these agents comprise at least inhibitors or agonists of groups 1 and 2, of groups 2 and 3, of groups 1 and 3, or preferably of groups 1, 2 and 3. In another preferred embodiment the kit comprises at least inhibitors or agonists of groups 1 and 4, of groups 2 and 4, or of groups 3 and 4. Preferably the agents according to the present invention or the compounds of the kit can be provided as a pharmaceutical preparation. The pharmaceutical preparation is e.g. a form of a topical or mucosal or intravenous or subcutaneous use.

Preferably the kit comprises a combination of antibodies recognizing 2, preferably 3, more preferred 4, even more preferred 5, also preferred 6, or more different epitopes on EphrinB3 or on Sema3A.

The pharmaceutical preparation may comprise pharmaceutical carriers, excipients or additives. The term carrier includes diluents, e.g. water, saline, excipients or vehicles with which the composition can be administered. Preferably, these compositions are provided as fluid compositions. Preferably the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. The agent may be in form of a pharmaceutically acceptable salt, for example a sodium salt. Further pharmaceutically acceptable salts include among others sodium, potassium, lithium and ammonium salts. The pH value of such a preparation is preferably in the physiologically exceptable range, preferably 5-9, more preferred at least 5,5, at least 6 and further preferred embodiments up to 8,5 or up to 8.

The agents can be provided in ready to use form or in stock amounts. The agents may be in liquid form such as solutions, emulsions or dispersions, or provided as solid compound, such as powders, tablets or capsules.

### Preferably the present invention is defined as follows:

Definition 1. The use of an agent capable of enhancing the PKA-p38-CREB pathway, suppressing the Fyn-RhoA-Rock and/or PKC-alpha-MARCKS pathways or Ephrin-B3 or Sema3A for the manufacture of a pharmaceutical composition for the treatment of damaged myelin sheaths, **characterized in that** the agent neutralizes the effect of inhibitors of the regeneration of damaged myelin sheaths mediated by OPCs and wherein the inhibitors are selected from myelin associated inhibitors, in particular Ephrin-B3 and inhibitors of the glial scar, in particular Sema3A.
Definition 2. An agent capable of enhancing the PKA-p38-CREB pathway, suppressing the Fyn-RhoA-Rock and/or PKC-alpha-MARCKS pathways or suppressing Ephrin-B3 or Sema3A for the treatment of damaged myelin sheaths, wherein the agent neutralizes the effect of inhibitors of the regeneration of damaged myelin sheaths by OPCs and the inhibitors are selected from myelin associated inhibitors, in particular Ephrin-B3 and inhibitors of the glial scar, in particular Sema3A.
Definition 3. Agent according to claim 2 being an enhancing agent of the PKA-p38-CREB pathway, preferably selected from cAMP agonists or phosphodiesterase-4 inhibitors, PKA agonists, p38 MAPK agonists or CREB agonists, even more preferred being selected from rolipram or db-cAMP.
Definition 4. Agent according to definition 2 being a suppressing agent of the Fyn-RhoA-Rock pathway and selected from ROCK inhibitors, Fyn agonists and, preferably being selected from RNAi of RhoA, RNAi of ROCK-II, or combinations thereof.
Definition 5. Agent according to definition 2 being a suppressing agent of the Fyn-RhoA-Rock pathway, preferably being a Rho inhibitor, more preferred being Fasudil or Y-27632, for the combined administration with an anti-inflammatory agent or with an oligodendrocyte precursor cell or a stem cell differentiating into oligodendrocytes.
Definition 6. Agent according to definition 2 being a suppressing agent of the PKC-alpha-MARCKS pathway, preferably a PKC-alpha inhibitor, more preferred being selected from RNAi of PKC-alpha, Gö-6976.
Definition 7. Agent according to definition 2 being one or a combination of anti-Ephrin-B3 antibodies or one or a combination of anti-Sema3A antibodies.
Definition 8. Agent according to definitions 2 to 7, **characterized in that** the agent neutralizes inhibitors of oligodendrocyte precursor cell differentiation to active oligodendrocytes.
Definition 9. Agent according to definitions 2 to 8, **characterized in that** the damaged myelin sheaths are associated with axon demyelination but not of axon destruction, preferably in the case of damage by inflammation, in particular in multiple sclerosis.
Definition 10. Agent according to definitions 2 to 9, **characterized in that** the damaged myelin sheaths are associated with disease or condition selected from being multiple sclerosis, stroke, nerve injury, in particular spinal cord injury, in central nervous system axons, of corticospinal tract axons, of dorsal column axons, acute disseminated encephalomyelitis, transverse myelitis, Alexander's disease, Canavan disease, Cockayne's syndrome, Pelizaeus-Merzbacher's disease, optic neuritis, hereditary leukencephalopathy, central pontine myelinosis, deep white matter Ischemia, progressive multifocal leukoencephalopathy, post-infectious encephalitis, demyelinating HIV encephalitis, demyelinating radiation injury, acquired toxic-metabolic disorders, posterior reversible encephalopathy syndrome, central pontine myelinolysis, demyelinating heredetary metabolic disorders, including leukodystrophies, in particular adrenoleukodystrophy, Krabbe's globoid cell leukodystrophy, metachromatic leukodystrophy.
Definition 11. Agent according to definitions 2 to 10, **characterized in that** the damaged myelin sheaths are of central nervous system axons, of corticospinal tract axons, of dorsal column axons, of optic nerve, optic tract axons or of axons in the penumbra after a stroke or after spinal cord injury.
Definition 12. Agent according to definitions 2 to 11, **characterized in that** the damage of the myelin sheaths is chronic or the result of a chronic disease, preferably wherein a glial scar is formed, in particular preferred a glial scar comprising Sema3A.
Definition 13. Agent according to definitions 2 to 12, **characterized in that** the agent is for administration after demyelination of a portion of the neuronal axons of a subject, which are in other respects undamaged.
Definition 14. Agent according to definitions 2 to 13, **characterized in that** the agent is for administration after 1 day, preferably after 3 days, more preferred after 1 week, even more preferred after 2 weeks, or even after one month of the onset of axon demyelination.
Definition 15. Agent according to definitions 2 to 13, **characterized in that** the agent is for administration after 1 day, preferably after 3 days, more preferred after 1 week, even more preferred after 2 weeks, or even after one month of the complete demyelination of at least a portion of the axons of a subject.
Definition 16. Agent according to definition 15, **characterized in that** the portion is 0.001%, preferably 0.01% even more preferred 0.1%, 1%, preferably 2%, more preferred 5%.
Definition 17. Agent according to definitions 2 to 16, selected from agents capable of enhancing the PKA-p38-CREB pathway or of suppressing the Fyn-RhoA-Rock and/or PKC-alpha-MARCKS pathways or suppressing Ephrin-B3 or Sema3A as defined in the preceding claims for administration together with at least one, preferably at least two additional agents suppressing a different pathway or molecular target of the pathways or target selected from Ephrin-B3 or Sema3A, as defined in the preceding claims.
Definition 18. Agent according to definition 17, **characterized in that** the agents for combined use enhance PKA-p38-CREB and suppress Fyn-RhoA-Rock, suppress Fyn-RhoA-Rock and PKC-alpha-MARCKS, enhance PKA-p38-CREB and suppress PKC-alpha-MARCKS pathways.
Definition 19. Agent according to any one of definitions 2 to 18, **characterized in that** the suppression is an inactivation of the pathways or target, preferably an inactivation of Ephrin-B3 or Sema3A or where enhancement includes the suppression of an endogenous suppressor of the pathways.
Definition 20. Agent according to definitions 2 to 19, for the combined administration with an anti-inflammatory agent, preferably a corticosteroid, cortisone, mitoxantron, natalizumab, interferon beta or glatiramer acetate.
Definition 21. Agent according to definitions 2 to 20 for the combined therapy with a pre-oligodendrocyte or oligodendrocyte precursor cell or embryonic or adult or olfactory bulb derived stem cell therapy.
Definition 22. A kit comprising at least two pharmaceutical compounds selected from at least two different groups of the following groups:
   group 1: cAMP agonists or phosphodiesterase-4 inhibitors, PKA agonists, p38 MAPK agonists or CREB agonists;
   group 2: ROCK inhibitors, Fyn agonists or Rho inhibitors;
   group 3: PKC-alpha inhibitors,
   group 4: Ephrin-B3 inhibitors or Sema3A inhibitors.
Definition 23. A kit comprising at least one pharmaceutical compound selected from the following groups:
   group 1: cAMP agonists or phosphodiesterase-4 inhibitors, PKA agonists, p38 MAPK agonists or CREB agonists;
   group 2: ROCK inhibitors, Fyn agonists or Rho inhibitors;
   group 3: PKC-alpha inhibitors,
   group 4: Ephrin-B3 inhibitors or Sema3A inhibitors and an anti-inflammatory agent, preferably for the combined administration for the treatment of myelin sheath damage.
Definition 24. A kit according to definitions 22 or 23, comprising at least inhibitors or agonists of groups 1 and 2, of groups 2 and 3, of groups 1 and 3, or preferably of groups 1, 2 and 3.
Definition 25. A kit according to one of definitions 22 to 24, comprising at least inhibitors or agonists of groups 1 and 4, of groups 2 and 4, or of groups 3 and 4.

The present invention is further exemplified by the following figures and examples, without being restricted thereto.
Figure 1: OPCs plated on myelin protein extracts (MPE, protein/cm²) display a concentration dependent down-regulation of relative MBP mRNA expression as assessed by qPCR after 3d culture in differentiation medium. (For this and subsequent figures: MPE: myelin protein extract; error bars: SEM).
Figure 2: Myelin Inhibitors impair Fyn-1/Y-418 phosphorylation in OPCs (a), immunoblot following anti-Fyn-1-immunoprecipitation and loading control (b). Myelin inhibitors also induce activation of RhoA (c). Autogradiograph of Rho assay (MPE: 40µg/cm²; PLL: poly-L-lysine; incubation in differentiation medium for 24h).
Figure 3: (a) Immunoblot of OPC lysates demonstrates that differentiating OPCs express ROCK-II. (b) Inhibition of ROCK by culturing OPCs on MPE with Fasudil (HA-1077) results in a more than 160% increase of differentiating cells after 48h culture in differentiation medium. (c) Whereas OPCs on MPE down-regulate 04 immunoreactivity (d) 04 expression is largely restored by treatment with HA-1077 (scale bar=30uM).
Figure 4: (a-f) MARCKS is a down-stream effector of PKC. While in control cells MARCKS expression is associated with the cell membrane, myelin inhibitors lead to a cytoplasmatic MARCKS presence. (g-n) Inhibition of PKC signaling in OPCs on MPE with BIM (g-j; max. mean increase 169 %) or Gö6976 (k-n; max. mean increase 269%) strongly induces OPC differentiation after 48 hours incubation in differentiation medium. (Scale bar: in a-c: 15.9µm, in d-f: 14.6µm in h-j, l-n: 30µm).

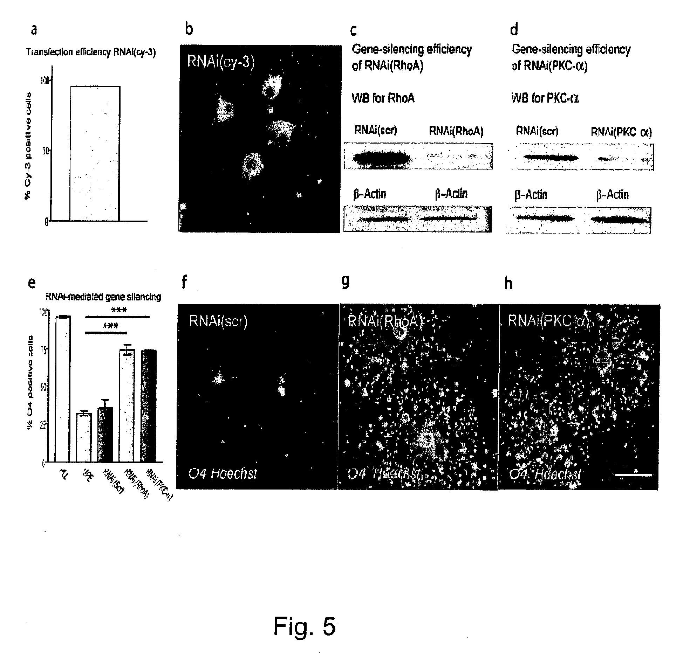

Figure 5: (a,b) Transfection efficiency of OPCs was monitored by transfecting cells with RNAi (cy-3) and assessing the proportion of cy-3-labelled cells after 48h in differentiation medium (n=3); in all experiments >95% of the cells were cy-3 positive. Immunoblots of OPC lysates after transfection with (c) RNAi for RhoA as well as (d) RNAi for PKC-a demonstrate downregulation of RhoA or PKC-α on protein level after 48h. (e) Silencing of RhoA or PKC-α results in a significant increase of 04 positive cells when cultured for 48 hours on MPE. (f) Whereas transfection with control RNAi (RNAi(scrambled, scr)) was not able to restore 04-immuroreactivity of OPCs on MPE (g,h) gene silencing induced a strong increase in the number of differentiating cells (scale bar=30µM).

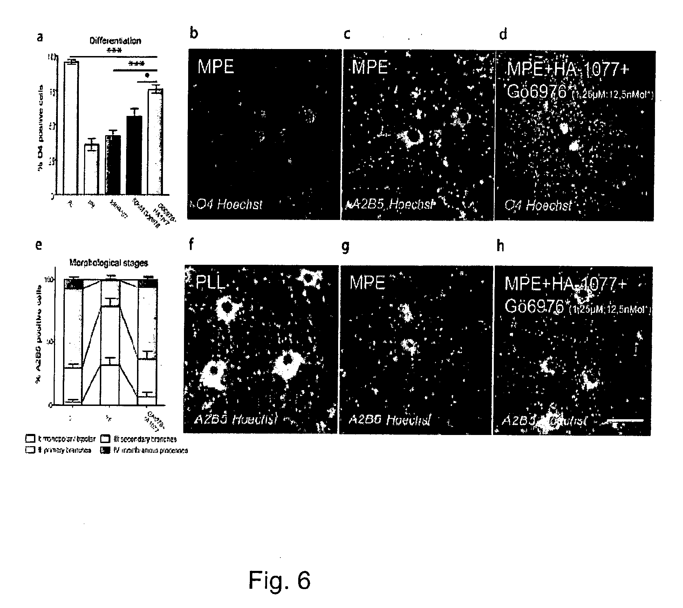

Figure 6: (a-c) Blocking PKC and ROCK-II simultaneously in OPCs plated on myelin by co-incubation with HA-1077 and Gö6976 induces an additional increase in the percentage of 04-positive cells as compared to single treatment after incubation in differentiation medium for 48h. (b) While MPE induces down-regulation of 04 immunoreactivity in OPCs (c: the same cells stained for A2B5), (d) 04 expression is restored after treatment with the pharmacological inhibitors. (e-g) The presence of MPE is associated with a reduction of the complexity of OPC processes and earlier morphological stages as compared to OPCs plated on control substrate, treatment with HA-1077 and GÖ6976 restores the presence of more mature phenotypes (scale bar=30µM).
Figure 7: (a,b) The number of TUNEL positive cells did not differ between OPCs on control substrate (PLL) or MPE. Neither siRNA mediated gene silencing nor pharmacological treatment induced changes in apoptosis.

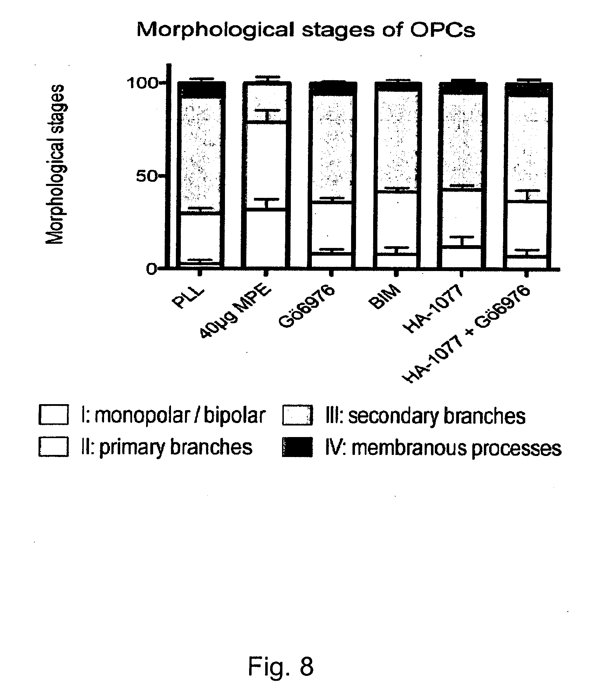

Figure 8. Morphological phenotypes of OPCs on control substrate, MPE, and after treatment with inhibitors, demonstrating that treatment restores the appearance of more mature phenotypes, which are inhibited by the presence of MPE.

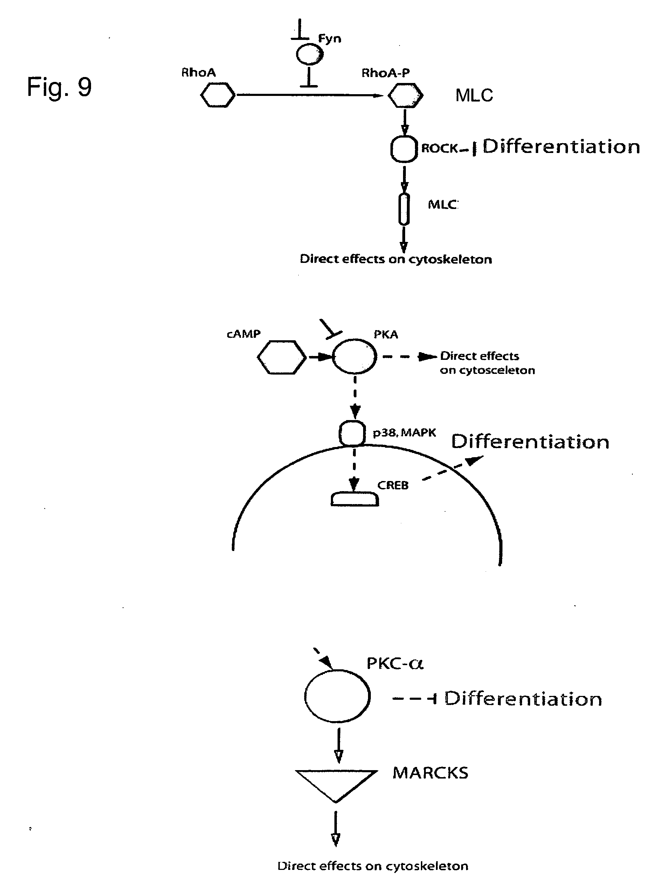

Figure 9: Scheme of the oligodendrocyte precursor cell differentiation by the PKA-p38-CREB, Fyn-RhoA-Rock, PKC-alpha-MARCKS pathways.
Figure 10(a): Effect of Ephrin-B3 on OPC differentiation: Ephrin-B3 induces a concentration-dependent inhibition of OPC differentiation (b): Neutralisation of myelin protein extract with Ephrin-B3 antibodies: Neutralising epitoptes of Ephrin B3 with antibodies or a combination of antibodies is able to overcome the differentiation block in OPCs induced by Ephrin B3; (c): Effect of Sema3A and OPC differentiation: the presence of Sema 3A induces a concentration-dependent inhibition of OPC differentiation;
Figure 11: RT-PCR for MBP mRNA confirms that myelin associated inhibitors are potent inhibitors of OPC differentiation;
Figure 12: p38 phosphorylation is down-regulated in the presence of myelin;
Figure 13: Myelin associated inhibitors inhibit CREB phoshorylation in OPCs;
Figure 14: Rolipram promotes OPC differentiation by inducing CREB phosphorylation;
Figure 15: Putrescine does not stimulate OPC differentiation in the presence of Myelin associated inhibitors demonstrating important differences to the process of axon regrowth.
Figure 16: Rank assessment of remyelination in vivo on resin-embedded semithin section demonstrates that remyelination is potently enhanced in rolipram treated animals (0.5 mg/kg/d) 14 days post lesion induction.
Figure 17: In situ hybridisation (ISH) for distinct markers demonstrates that Rolipram treatment following induction of demyelination in Sprague-Dawley rats results in (a) maintained levels of PDGFR-α positive OPCs at day 14, (b) an increase of active OPCs at day 7 assessed by Nkx2.2, and (c) a strong increase in PLP-pos. cells corresponding to mature oligodendrocytes at day 14 as compared to controls. Furthermore, ISH for the macrophage marker MsrB demonstrates that treatment did not affect the immune response(d).
Figure 18: Protein extracts of myelin membrane preparations inhibit OPC differentiation in vitro.
Figure 19: cAMP is able to partially overcome the inhibition of OPC differentiation by myelin in vitro.
Figure 20: The Phosphodiesterase inhibitor rolipram is able to restore OPC differentiation on myelin in vitro.

### EXAMPLES

### Example 1: Material and Methods Preparation and Purification of oligodendrocyte precursor cells (OPCs)

Primary cultures of oligodendrocyte precursor cells were isolated from PO-P2 neonatal Sprague-Dawley rat forebrains following a Standard protocol (McCarthy et al., J Cell Biol 85 (1980): 890-902) that were adapted for the present purpose (Syed *et al.* 2008, supra). In brief, hemispheres were stripped free of meninges, after a digestion step the cells were plated into cell culture flasks and mixed glia cultures were grown for approx. 10 days in DMEM medium supplemented with 10% PCS at 37°C and 7,5% CO₂. To remove the loosely attached microglia the flasks were shaken for 1 hour at 260rpm on an orbital shaker before being shaken at 260rpm overnight to dislodge the loosely attached oligodendrocyte precursors. OPCs were further purified from contaminating microglia by a differential adhesion step. Subsequently OPCs were plated onto polylysine (PLL)-coated or Substrate coated dishes. To maintain cells at an early precursor stage PDGF-AA (Pepro Tech) and FGF (Pepro Tech) were added (10ng/ml) to SATOs medium. To induce differentiation cells were incubated in SATOs medium supplemented with 0.5% FCS. The purity of each culture was monitored following OPC purification by immunocytochemistry and only cultures with >94% purity were used. In the cultures minor contaminations of microglia that can be detected by isolectin staining and which amount to 4-5% of the cells, as well as by astrocytes (detectable by GFAP) and fibroblasts with distinct morphology, which account for about 1-2% of the cells are present.

### Preparation of myelin membrane substrates and myelin protein extracts

Myelin was purified by two rounds of discontinuous density gradient centrifugation and osmotic disintegration. Total brains of young Sprague-Dawley rats were homogenized mechanically in ice-cold 0.32M sucrose using a mechanical blender (Ultra-Turrax; IKA, T18 basic, Germany) (Sucrose was dissolved in sterile 2.5mM Tris/HCI, pH 7.0, to form 0.25, 0.32, and 0.88M Solutions). The homogenized brains were diluted to a final 0.25M sucrose solution and pelleted in an ultra-centrifuge (55,000 x g, 4°C, 15 min). The pellet was re-suspended in 0.88M sucrose solution and overlaid with 0.25M sucrose. After an ultracentrifugation step (100,000 x g, 4°C, 1 h), the Interface was collected and washed in 30 ml of distilled H₂O (55,000 x g, 4°C, 10 min). The pellet was re-suspended in dH₂O and incubated for 60 min on ice for osmotic disintegration. After centrifugation (55,000 x g, 4°C, 10 min), the flotation step was repeated. The Interface was collected and washed twice in dH₂O (55,000 x g, 4°C, 10 min), and the pellet was stored at -80°C until Isolation of myelin protein extracts.

To prepare myelin protein extract (MPE) the pellets were re-suspended in 1% N-octyl β-D-glucopyranoside, 0.2M Sodiumphosphate pH 6.8, 0.1 M Na2SO₄ and 1mM EDTA and incubated at 23°C for 2 hours. Following an ultracentrifugation step (100,000 x g, 18°C, 30 min) the supernatants were collected and stored at -20°C until further usage.

### Isolation of liver membrane fractions

0.85M and 1.23M sucrose in 2.5mM Tris/HCI was prepared as outlined above. The liver of a young adult Sprague-Dawley rat was extracted and homogenised in 0.85M sucrose (using an Ultra-Turrax). The homogenate was layered onto a 1.23M sucrose solution. After centrifugation (100,000g, 4°C, 1h) the Interface was collected and washed in dH₂O (55,000g, 4°C, 10min). Finally, the pellet was re-suspended in an equal volume of 1xPBS containing 1% octyglucoside and 1 mM EDTA. After incubation at 25°C for 2 hours in a shaker the sample was ultracentrifuged at 100,000g for 30 min and finally, the supernatant was stored at -20°C until further use. The protein concentration was estimated by BCA assay (Pierce) (Kotter *et al*. 2006).

### Immunocytochemistry

The pooled OPCs harvested as outlined above were seeded at the same density of 20000 cells per well into polylysine (PLL)-coated 8-well chamber slides. Cells were differentiated for 48 hours and subsequently fixed with 4% paraformaldehyde in PBS, permeabilised and blocked with 0.3% Triton X-100 and 10% NGS. To assess the differentiation state of OPCs the cells were incubated with 04 antibody (1:100; Chemicon) for 1 hour in the presence of 0.1% Triton X-100 and 2% NGS, washed, and incubated for another 1 hour with the appropriate fluorescent secondary antibody (Cy3-conjugated antibody 1:100; Jackson Immuno Research) and the nuclei were stained using DAPI (Robinson et al., Dev Biol 216 (1999): 359-368; Syed et al. 2008, supra). It is important to note that permeabilisation of cells results in a punctate representation of the extracellular antigen 04 (Reynolds et al., Curr Opin Biotechnol 4 (1993): 734-738; Weiss et al., J Neurosci 16 (1996): 7599-7609; Syed et al. 2008, supra). Under an Olympus X51 fluorescent microscope using a triple-filter the percentage of O4-positive cells in relation to >100 DAPI-stained nuclei in randomly selected eye fields for each experimental condition were determined. To establish the purity of the OPC cultures immunocytochemistry for A2B5 (1:100; Chemicon) was performed according to the same principles and the percentage of A2B5+ cells to >100 DAPI-stained nuclei in randomly selected eye fields was determined. Only cell cultures with >94% A2B5-pos. cells were used for the present study. To assess the morphological phenotype of OPCs, A2B5+-cells were categorised according to the following criteria: stage 1: mono/bipolar; stage II: multipolar, primary branched; stage III: multipolar, secondary branched; stage IV: secondary branched cells with membranous processes.

### qPCR for MBP vs β-2-microglobulin

Total RNA of OPCs grown on PLL control or MPE Substrates with various concentrations (0.4µg MPE, 4µg MPE and 40µg MPE) was harvested after the cells were differentiated for three days using RNeasy Mini Kit (Quiagen) according to the manufacturer's instruction. 1^{st} Strand cDNA synthesis kit for RT-PCR (Roche Applied Science) was used for reverse transcription of 500ng RNA (each sample) according to the manufacturer's instructions. q-PCR was performed using Taqman gene expression assays for MBP (ABI, Rn 00566745_M1) and β-2-microglobulin (β2-MG) (ABI, Rn 00560865). Three independent experiments were conducted and all reactions were performed in triplicates on a 7500 Fast Real-Time PCR System. Semi-quantitative mRNA expression levels were calculated with the 7500 Fast System Software (ABI, Foster City, CA).

### Pharmacological Inhibition of ROCK-II and PKC

To examine whether ROCK-II and PKC Signal transduction is implicated in the myelin mediated differentiation block, pharmacological inhibitors were added to the culture medium immediately after cell seeding. The selective PKC-inhibitor Gö6976 (12-(2-cyanoethyl)-6,7,12,13-tetrahydro-13-methyl-5-oxo-5H-indolo[2,3-a]pyrrollo[3,4-c]carbazole; CAS-number: 136194-77-9), specific for PKC-α and PKC-β, and BIM (Ro 31-8220, Bisindolylmaleimide IX; CAS-number: 125314-64-9), which selectively affects all PKC isoforms, were used to block PKC-signalling. To inhibit ROCK-II, an orally available drug currently evaluated for the treatment of vascular disease named Fasudil (HA-1077 dihydrochloride; 1-(5-lsoquinolinesulfonyl)-1H-hexahydro-1,4-diazepine 2 HCI; CAS-number: 103745-39-7) has been used. Fasudil has the potential of interfering with Rho kinase, PRK2, MSK1, PKA, PKG, S6K1, MAPKAP-K1 b, MLCK, and CaMKII signalling. Gö6976 and BIM were solubilised in DMSO. OPCs plated on different substrates were differentiated for 48 hours and subsequently fixed for immunocytochemistry. To quantify differentiation the ratio of 04 positive cells and the number of Hoechst-positive nuclei was determined by cell counts using an Olympus IX 51 fluorescence microscope. A minimum of 200 cells were counted for each experiment and a minimum of 3 independent experiments were conducted.

### siRNA-based down-regulation of RhoA and PKC-α

Ten days after plating mixed glial cultures were transfected with siRNAs (Applied Bieosystems, AM4621: Silencer Cy3 Neg Control #1 siRNA 5nmol) for PKC-α, RhoA, negative control siRNA and Cy3 labelled negative control (Applied Biosystems) using Lipofectamine 2000 (Invitrogen) at a concentration of 100nM according to the manufacturers protocol. 24h later OPCs were harvested as outlined above. To determine the knock-down efficiency of the targeted genes, western blot analyses were performed for RhoA (Upstate) or PKC-α (Cell Signaling). OPC differentiation was assessed as outlined above 48h after plating cells onto the experimental Substrates; at least 3 independent experiments were conducted for each condition.

### TUNEL assay

Fragmented DNA was detected by incorporation of biotinylated nucleotides at the 3'-OH DNA ends using Terminal Deoxynucleotidyl Transferase recombinant (rTdT) enzyme according to the manufacturer's instruction (Promega). Stained cells were visualized by light microscopy and the percentage of apoptotic nuclei was determined.

### Western blot analysis

OPCs differentiated for 24 hours and 9 days on PLL were lysed in a buffer containing 10mMTris, pH 7.4, 100mM NaCl, 1mM EDTA, 1mM EGTA, 1mM NaF, 20mM Na₄P₂O₇, 2mM Na₃VO₄ 0.1% SDS, 0.5% Sodium Deoxcholate, 1%Triton-X100, 10% Glycerol, 1mM PMSF, 60µg/ml Aprotinin, 10µg/ml Leupeptin, 1µg/ml Pepstatin. After high speed centrifugation, protein concentration was measured using BCA™ protein assay (Pierce) and 15µg were loaded on SDS-PAGE for separation. Western blot was conducted using a Multiphor II Electrophoresis System (Amersham Biosciences) at 200mA for 1.30 hours. After blocking with 5% BSA in TBS-T (0.1% Triton X 100 in TBS) for one hour at room temperature, the blots were incubated with ROCK 11/ROKα (BD Biosciences); RhoA (Upstate) or PKC-α (Cell Signaling) antibodies over night following incubation with horseradish-peroxidase conjugated secondary antibody (Amersham Biosciences) for one hour. The immunoreactive bands were detected by ECL™ chemiluminescence (Amersham Biosciences) reagent on a film. To confirm the presence of proteins identified by MS analysis in the purified inhibitory fractions, 10µg of protein was separated using 12% Novex gel (Invitrogen). MPE was used as positive control and protein extracts of liver membrane preparation as negative control. Following SDS-PAGE, the protein was then transferred to PVDF membrane using an Xcell II Blot Module (Invitrogen). After blocking with 5% BSA in TBS-T (0.1% Triton X 100 in TBS) for one hour at room temperature, it was probed overnight at 4°C with the following antibodies: MAG (Chemicon), PLP (kindly provided by Lees MB), MBP (DAKO). Following a wash step the membranes were incubated with the appropriate horseradish-peroxidase conjugated secondary antibody (Amersham Biosciences) for one hour. The immunoreactive bands were detected by ECL™ chemiluminescence (Amersham Biosciences) reagent on a film.

### Evaluation of Fyn activation

Cells were washed twice with ice cold PBS before being lysed in 10mM Tris, 100mM NaCl, 1mM EDTA, 1mM EGTA, 1mM NaF, 2mM Na₃VO₄, 0.1% Triton, 10% Glycerol, 1mM PMSF, 0.5% Na-Deoxycholate, 20mM Na₄P₂O₇ and protease Inhibitor (Roche). Cell lysates were centrifuged (1h, 3200rpm, 4°C) and the protein concentration was estimated by BCA™ assay (Pierce). The lysates were pre-cleared with A/G Plus Agarose beads (Santa Cruz) for 30 min at 4°C and incubated with total Fyn antibody (Santa Cruz) for 2 hours in a rotary mixer following addition of A/G Plus Agarose beads for overnight incubation. Before performing SDS-PAGE and western blot, the immune complexes were washed extensively with NP-40 buffer and PBS. The precipitates were re-suspended in sample buffer, heated to 95°C for 5 min, and subjected to SDS-PAGE. Proteins were transferred to a PVDF membrane, blocked with bovine serum albumin and incubated with Y-418-phospho-Src (Biosource) antibody. Finally, a horseradish-peroxidase conjugated secondary antibody was added and detected by ECL™ chemiluminescence (Amersham Biosciences) reagent on film. The optical density of the bands was estimated with NIH-lmage J (free download at http://rsb.info.nih.gov/ij/) on digitised images.

### RhoA GTPase activity assay

For the detection of active RhoA, OPCs were lysed (125mM HEPES, pH 7.5, 750mM NaCl, 5% Igepal CA-630, 50mM MgCI₂, 5mM EDTA, 10% Glycerol, 25mM NaF, 1mM Na₃VO₄) according to the manufactures instructions (Upstate) and active GTP-Rho was precipitated by the use of beads specific for the GST-binding domain (RBD) of rhotekin. After removing cell debris, the lysates were incubated with Rho Assay Reagent Slurry which specifically binds Rho-GTP and not Rho-GDP (30 min, 4°C). Beads were then washed with Mg²+ Lysis/Wash Buffer and bound material was eluted with 25µI 2x Laemmli sample buffer, boiled for 5 min, resolved by SDS-PAGE, and immunoblotted using mouse anti-RhoA antibody (3µg/ml). Peroxidase-conjugated anti-mouse IgGs (Amersham Biosciences) were used as secondary antibodies. Immunoreactive proteins were visualized using ECL™ detection System (Amersham Biosciences). Densitometric analysis was performed using NIH-lmage J software.

### Statistical analysis

For all studies at least 3 independent experiments (n as detailed in results) were conducted and statistically assessed using Graph Pad Prism Software (Graph Pad, San Diego, CA). To test the concentration dependent Inhibition of OPC differentiation on MPE, and the effect of various concentrations of pharmacological Inhibitors, one way ANOVA followed by Dunett's Multiple Comparison test was used. ROD values from western blot following immunoprecipitation or pull-down assay were compared with Student-T test.

### Example 2: Soluble myelin molecules inhibit the differentiation of OPCs

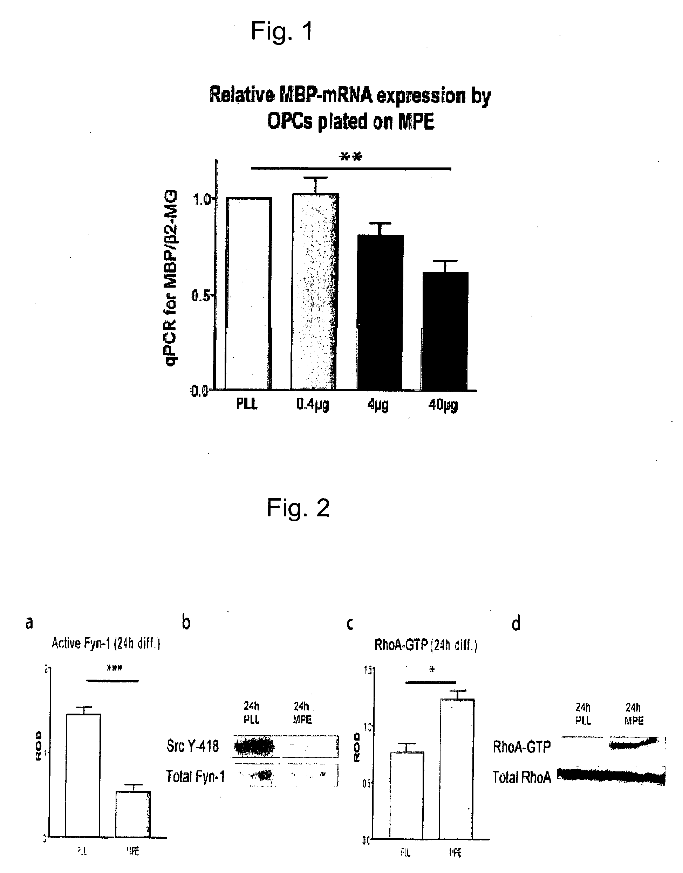

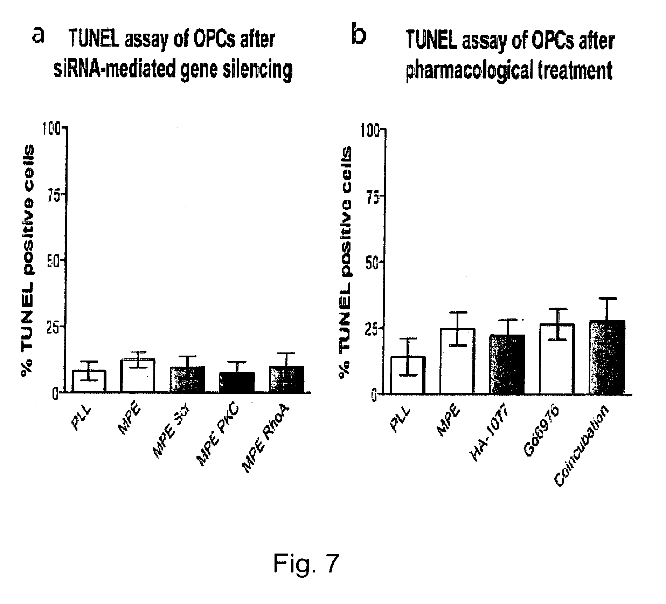

To establish an *in vitro* assay for assessing the effects of myelin on OPC differentiation, an approach previously reported by Robinson and Miller was adapted and neonatal OPCs were plated on myelin Substrates produced by incubation of myelin preparations on poly-L-lysine covered culture dishes (Robinson et al., 1999, supra). The differentiation of oligodendrocyte lineage cells (OLCs) *in vitro* is characterised by distinct morphological and immunological features. Antibodies against 04 identify late OPCs by reacting with a sulfated glycolipid antigen named POA (Proligodendrocyte Antigen), while the same antibody also recognises terminally differentiated OLCs by reacting with sulfated galactosylcerebroside (Bansal et al., J Neurosci Res 24 (1989): 548-557). The differentiation block mediated by presence of myelin is manifested as a reduction in O4-expression by OPCs (Robinson et al., 1999, supra). In a previous study it was shown that myelin protein extracts (MPE) prepared with n-octyl-glycoside induce a concentration-dependent Inhibition of differentiation in OPCs cultured in differentiation medium for 48h similar to that observed with crude myelin membrane preparations (Syed *et al*. 2008, supra) The Inhibition of OPC differentiation is not only reflected by a reduction of 04 expression but also by a corresponding down-regulation of mRNA expression for myelin basic protein (MBP), a late marker of mature oligodendrocytes, as assessed by qPCR (fig.1; n=3;). The presence of myelin was associated with a reduction in the complexity of OPC processes resulting in an increase of early OPC phenotypes with bipolar (stage 1) morphology or primary branches (stage II) and a consecutive reduction of OPCs with secondary branched or membranous processes (stage III/IV) (fig. 6) (1-way ANOVA p<0.0001). Previous data (Robinson et al., 1999, supra) was confirmed demonstrating that no changes occurred in the rate of cell proliferation as assessed by BrdU staining (no BrdU-pos. cells were detected in the experimental groups) or apoptosis assessed by TUNEL assay, indicating that differences in the proportion of O4-positive cells reflect changes of cell differentiation and that these were not caused by an increase of OPC proliferation or differences in the rate apoptosis (fig. 7).

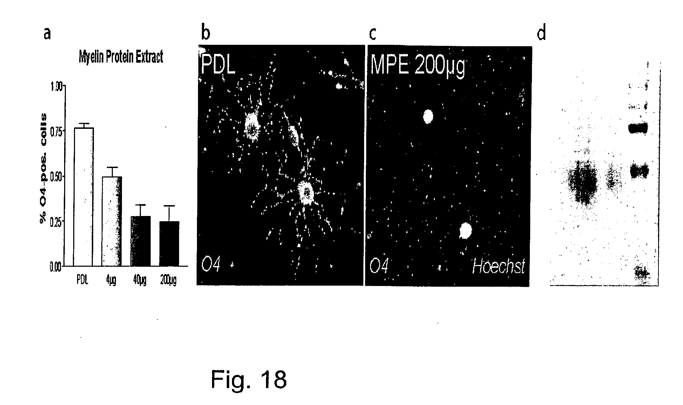

With reference to Fig. 18 it was demonstrated that myelin selectively arrests OPC differentiation. This has been verified by down-regulation of 04 in vitro. OPCs plated on myelin protein extracts, prepared with 1% octylglucoside (1ml/mg protein) in 0.2M phosphate buffer (pH 6.8) containing 0.1M Na₂SO₄, 1mM EDTA, 1mM DTT, and protease inhibitors, display a similar concentration-dependent down-regulation of O4-expression as when OPCs are plated on crude myelin membrane preparations (fig. 18 a). Whereas a large proportion of OPCs on control substrate are 04 immunoreactive (fig. 18b), 04 expression of OPCs plated on myelin proteins is suppressed (fig. 18 c). Coomassie-stained SDS-PAGE shows proteins extracted from myelin preparations with octylglucoside (fig 18 d).

### Example 3: Myelin impairs activation of the Src family tyrosine kinase Fyn-1

To investigate the basis of the myelin mediated Inhibition of OPC differentiation the activation of Src family tyrosine kinase Fyn-1 was examined. Several lines of evidence implicate an important role for Fyn-1 in the formation of myelin sheaths: first, the myelin content found in brains of fyn-deficient mice is reduced, and second, Fyn-1 can be co-immunoprecipitated from brain lysates with antibodies to myelin-associated glycoprotein (MAG). More recent data suggest that activation of Fyn-1 is one of the earliest events triggered in differentiating OPCs and that Fyn-1 tyrosine kinase regulates process extension and myelin sheath formation. Protein extracts from OPCs, plated on MPE cultured in differentiation medium for 24h, were compared with protein extracts from OPCs plated on control substrates under the same conditions by immunoprecipitation with anti-Fyn-1 and immunoblotting with anti-Src Y-418, to detect phosphorylation at the activation site of Fyn-1. The results demonstrated a clear impairment of Fyn-1 activation in cells plated on MPE (n=4; t-test p=0.0003; fig.2 a,b). In OPCs plated on control Substrate phosphorylation of Fyn-1 increased over time as previously reported.

### Example 4: Myelin molecules mediale inhibitory Signals via activation of RhoA

Fyn-1 kinase regulates the activity of the small GTPase RhoA, which, along with other Rho GTPAse subfamily members, is an important regulator of oligodendrocyte morphology. Over-expression of dominant-negative RhoA causes hyperextension of oligodendrocyte processes while a reduction of active RhoA-GTP is necessary for successful oligodendrocyte differentiation. To assess activation of RhoA in OPCs cultured on MPE and control substrates for 24 hours in differentiation medium, a RhoA-GTP-pull down assay followed by western blot for RhoA was performed. These data show that the presence of myelin iInhibitors induces an increase of GTP bound RhoA (n=3; t-test: p=0.0113; fig.2 c,d). To assess the functional role of RhoA in mediating inhibitory effects of MAI to OPC differentiation, OPCs were transfected with siRNA specifically directed against RhoA. Cells were transfected with high efficiency (>95%). This induced a marked down-regulation of the expression of RhoA as assessed by western blot. The reduction of RhoA powerfully triggered OPC differentiation in the presence of MPE (fig. 5; n=3; mean increase = 233%; ANOVA: p<0.0001, Dunnett's post test MPE vs. siRNA: p<0.0001) confirming the importance of RhoA in mediating the inhibitory effects as well as indicating that these can be beneficially modulated by inhibiting RhoA signalling in OPCs.

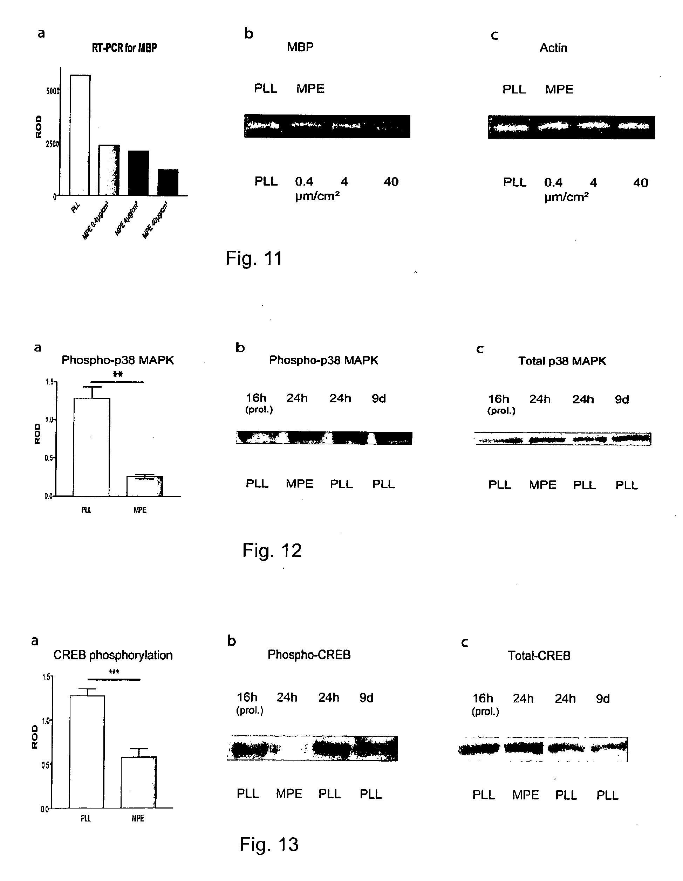

The differentiation of oligodendrocyte lineage cells (OLCs) in vitro is characterised by distinct morphological and immunological features. Antibodies against 04 identify late OPCs by reacting with a sulfated glycolipid antigen named POA (Proligodendrocyte Antigen), while the same antibody also recognises terminally differentiated OLCs in which case O4-antibody reacts with sulfated galactosylcerebroside. The differentiation block mediated by presence of myelin is manifested as a reduction in O4-expression by OPCs. To confirm that the inhibition of 04 expression is a marker of OPC differentiation, MBP expression has been assessed by RT-PCR in OPCs. The results indicate that the presence of MAI induce a concentration-dependent inhibition of MBP mRNA expression (fig. 11, a-c). RT-PCR for MBP mRNA confirms that MAI are potent inhibitors of OPC differentiation.

### Example 5: Inhibition of ROCK-II induces differentiation of OPCs in the presence of myelin

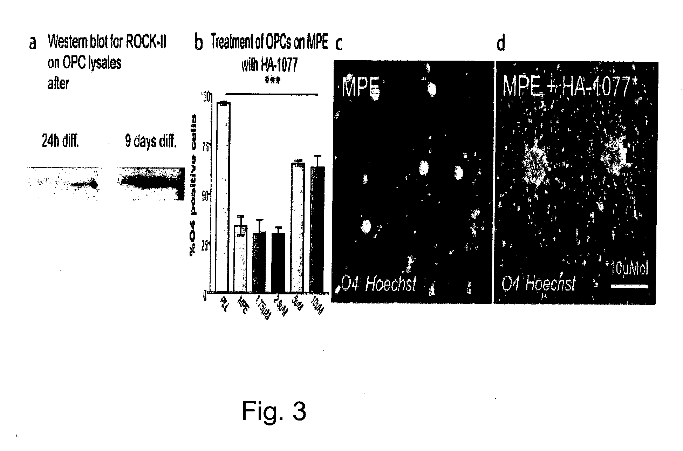

Rho kinases (ROCK) are important effector proteins of RhoA and phosphorylate a number of downstream molecules that regulate actin filaments. Since ROCK-II is the isoform predominantly expressed in the CNS, its participation in transducing myelin derived inhibitory Signals in OPCs was hypothesised. First ROCK-II expression in OPCs was confirmed by western blot analysis and then its functional role was examined by plating OPCs on MPE and adding different concentrations of the ROKa/ROCK 11-inhibitor HA-1077 (Fasudil) to the differentiation medium (fig.3). Treatment with HA-1077 for 48 hours resulted in a dramatic increase in the number of differentiating OPCs (n=4; mean increase at 5µM = 174%; ANOVA: p<0.0001, Dunnett's post tests MPE vs. 5µM and 10µM: p<0.001). To exclude changes in cell survival TUNEL assays were conducted which showed no significant differences between OPCs plated on PLL and cells plated on MPE treated with HA-1077 (fig. 7). Thus, it was concluded that RhoA-ROCK signalling plays an important role in mediating myelin derived inhibitory effects in differentiating OPCs that can be beneficially modulated by the use of pharmacological inhibitors and siRNA-mediated gene silencing.

### Example 6: PKC Inhibitors promote OPC differentiation in the presence of myelin Inhibitors

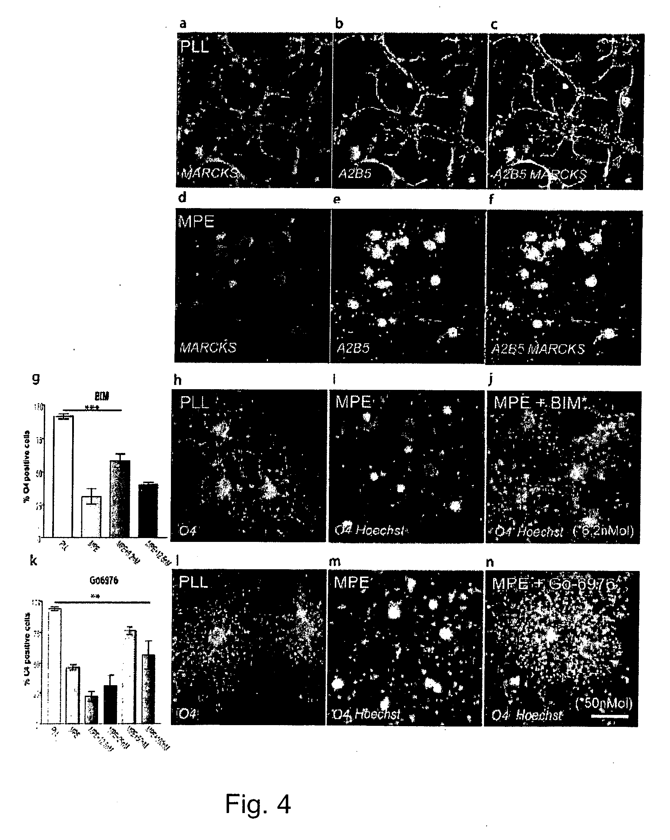

Myelin Inhibitors retain MARCKS within the cytosolic compartment. Protein kinase C (PKC) has also been implicated in OPC differentiation, since PKC activation, mimicked by the phorbol ester PMA, inhibits OPC differentiation, while at late stages of OPC differentiation PKC may also facilitate process extension and expression of myelin proteins. Myristoylated, alanine-rich C-kinase Substrate (MARCKS) is a Substrate of PKC that has been used as an indirect marker for PKC activation in OPCs. Inhibitory PKC mediated signalling is associated with phosphorylation and membrane-to-cytosol translocation of MARCKS and may cause disorganisation of the cytoskeleton and redistribution of actin filaments. MARCKS has been implicated in several cellular processes such as secretion, phagocytosis, cell motility, membrane traffic, growth suppression, and regulation of the cell cycle as well as OPC differentiation. Phosphorylated MARCKS is translocated from the plasma membrane to the cytosol. To detect changes in the intracellular distribution of MARCKS, OPCs plated on MPE and control-PLL stained for A2B5 and MARCKS were compared using a laser scanning microscope (fig.4 a-f). In differentiating OPCs MARCKS was distributed to the cell membrane, whereas in OPCs in which differentiation was inhibited by the presence of MPE a clear cytosolic presence of MARCKS was detected, supporting that PKC is activated by myelin and that myelin Inhibitors modulate cytoskeletal dynamics by MARCKS.

In order to test whether Inhibition of PKC signalling is able to modulate the myelin-mediated differentiation block, OPCs plated on myelin were treated with the two selective PKC Inhibitors, BIM (Bisindolylmaleimide IX, Methasulfonate Salt), and Gö6976. It was found that treatment with BIM (n=4; mean increase at 6.25nM = 169%; ANOVA: p<0.0001, Dunnett's post tests MPE vs. 6.25nM: p<0.001; 12.5nM: p<0.05) and even more so with GÖ6976 (n=3; mean increase at 50nM = 267%; ANOVA: p<0.0001, Dunnett's post test MPE vs. 25nM: p<0.05; 50nM: p<0.001) was able to stimulate OPC differentiation in the presence of myelin (fig.4 g-n). TUNEL assays showed no differences in the rate of apoptosis between control cells and OPCs plated on myelin treated with Gö-6976 (fig. 7b). As the use of pharmacological inhibitors entails the risk of regulating cascades other than the targeted, siRNA was used to down-regulate PKC-a expression in OPCs plated on myelin (fig.5). SiRNA-mediated silencing of PKC-α was successful and cells transfected displayed a marked decrease of PKC-α at protein level. The reduction of PKC-α potently induced OPC differentiation on MPE confirming the validity of the pharmacological approach (n=3; mean increase = 232%; ANOVA: p<0.0001, Dunnett's post test MPE vs. siRNA: p<0.0001) (fig. 5).

### Example 7: P38-phosphorylation is down-regulated in the presence of myelin

An important effector of cAMP in differentiating OPCs the transcription factor CREB which in turn plays a functional role with respect to cAMP induced differentiation. CREB activation can be mediated by phosphorylation of p38. To assess whether myelin induces an inhibition of p38 activation immunoprecipitations for p38 followed by Western blot for phospho-p38 were performed. The data obtained confirm that MAI inhibit phosphorylation of p38 (fig. 12, a-c).

As outlined above, CREB phosphorylation is an important event in the process of OPC differentiation. Assessment on the basis of immunoprecipitation for CREB followed by Western blot for phospho-CREB revealed that the presence of MAI impair activation of CREB (fig. 13, a-c).

### Example 8: cAMP signalling promotes OPC differentiation in vitro

Early in vitro studies have demonstrated that cAMP plays an important role during the differentiation of OPCs, accelerating their differentiation rate and inducing expression of myelin proteins. Similarly, activation of adenylate cyclase, which is capable of generating cAMP, induces oligodendrocyte differentiation. In OPCs PKA activation mediates CREB phosphorylation which in turn plays a functional role with respect to cAMP induced differentiation. Although the addition of the polyamine putrescine per se does not stimulate OPC differentiation, the absence of this polyamine has been shown to impair their maturation.

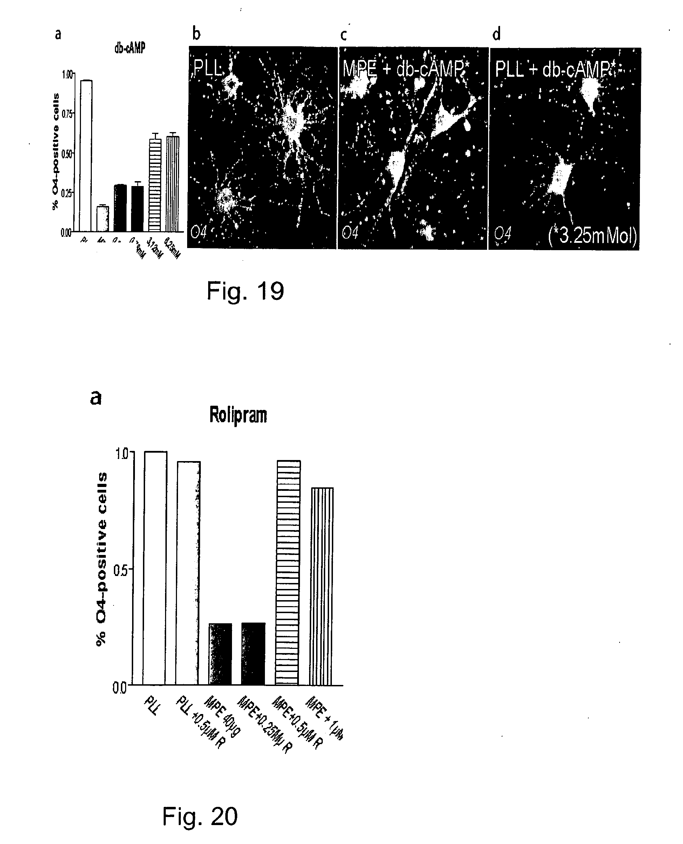

It has now been found that cAMP is able to at least partially overcome the inhibition of OPC differentiation by myelin in vitro. An increase of intracellular cAMP can be mimicked by addition of the membrane permeable analogue db-cAMP. It has been demonstrated that db-cAMP can promote OPC differentiation in vitro and induce the expression of myelin proteins. In order to test whether cAMP can overcome the inhibitory effects of myelin on OPC differentiation, OPCs plated on myelin protein extract where incubated with different concentrations of db-cAMP. The results indicate that db-cAMP can partially overcome the myelin mediated differentiation block and restore OPC differentiation (fig. 19 a-d).

### Example 9: Rolipram is able to promote remyelination in vivo

An alternative way of increasing intracellular cAMP is the inhibition of phosphodiesterase by rolipram. One of the advantages of rolipram is its ease at which it can be administered in vivo. To determine whether rolipram is able to modulate the inhibition of OPC differentiation by myelin, rolipram was added to the medium. At at a concentration of 0.5µM rolipram was able to completely restore the differentiation capability of OPCs plated on MPE. The effects of rolipram on OPC differentiation in the presence of myelin are paralleled by the effects of rolipram on axon outgrowth, a process that is equally inhibited by the presence of myelin (fig. 20).

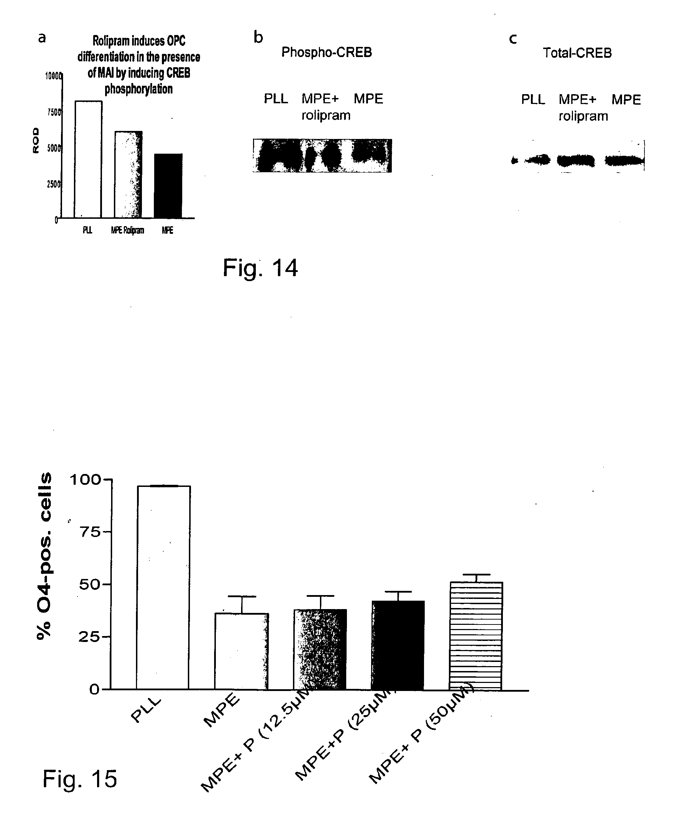

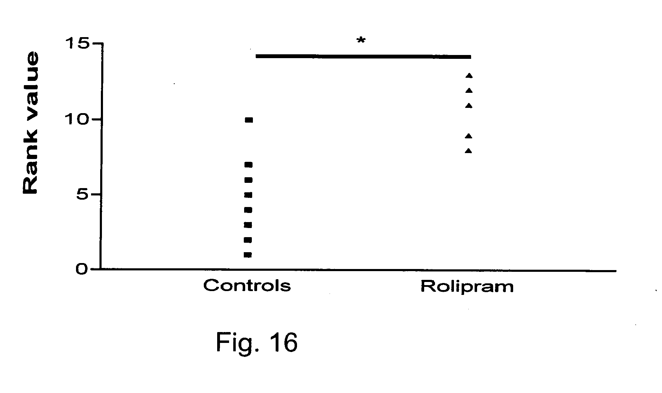

Further evidence for the importance of CREB activation to overcome the myelin mediated differentiation block derives from the fact that treatment of OPCs with the phosphodiesterase inhibitor rolipram, which is able to induce OPC differentiation in the presence of MAI in vitro, restores CREB activation in OPCs (fig. 14). The in vitro finding that rolipram is able to promote OPC differentiation in the presence of MAI was further developed to confirm remyelination in vivo. For this purpose demyelinating lesions were induced by topic injection of ethidium bromide into white matter tracts of young adult Sprague Dawley rats, and rolipram was applied subcutaneously by continuous osmotic pumps. 0,5mg/KG/d of rolipram was used in the first set of experiments that assessed the rate of remyelination. The preliminary results obtained by blind ranking of the extent of remyelination on toluidine blue stained semithin resin sections indicated that rolipram is able to promote remyelination in-vivo. This is the first experiment that succeeded in enhancing the efficiency of remyelination in this model (Fig. 16).

To validate the finding that administration of rolipram promotes CNS remyelination, the experiments were repeated as follows. EB lesions were induced in Sprague-Dawley rats, and rats were treated either with rolipram (0,5mg/KG/d) or PBS vehicle administered via s.c. osmotic pumps implanted 3 days post lesion induction. Animals were sacrificed at day 7 and day 14 post lesion induction and the tissue processed for in situ hybridization. The lesion area was visualized and measured on digitized solochrome cyanine stained sections and the number of cells within the lesion area as detected by ISH was determined.

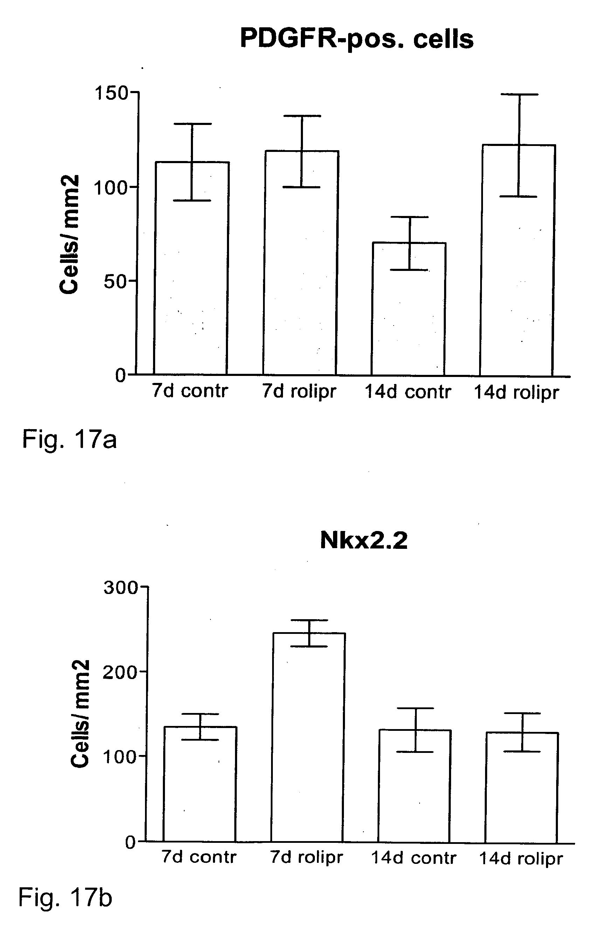

PDGFR-α, a general marker for OPCs, was used to determine the presence of OPCs in the lesions. It was found that the number of OPCs was similar in both groups at day 7 when a peak in the presence of OPCs in demyelinating lesions is to be expected. While the number of OPCs in control animals decreased after 14 days, the number of OPCs in rolipram treated animals remained elevated and comparable to levels at 7 days (Fig. 17a).

Nkx2.2 can be used to detect activated OPCs within remyelinating lesions. ISH for Nkx2.2 demonstrated that rolipram treatment effectively increased the number of active OPCs at the beginning of remyelination at day 7. At later stages, when remyelination has progressed further, the number of Nkx2.2 positivecells reached levels of control animals (Fig. 17b).

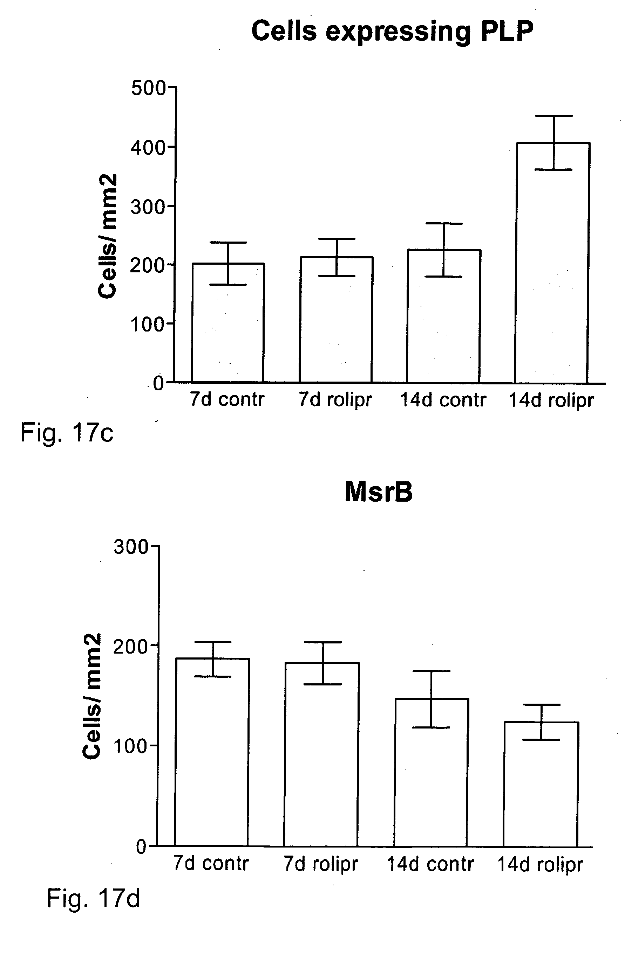

To detect mature oligodendrocytes, in situ hybridization for PLP was conducted. The extent of remyelination in control animals at 14 days is minimal. As to be expected in control animals the number of PLP positive cells remained unchanged at day 7 and day 14. Conversely, rolipram treatment induced a significant increase in the number of mature oligodendrocytes (Fig. 17c). These results validate the results obtained by blind-ranking resing sections for the extent of remyelination observed.

Finally, ISH for MRSB, a marker for macrophages, was used to determine whether rolipram treatment affects the immune response. There were no differences detected in the levels of rolipram treated vs control animals at day 7 and day 14 indicating that the rolipram selectively promotes the differentiation of OPCs into mature oligodendrocytes (Fig. 17d).

### Example 10: Putrescine is not able to induce OPC differentiation in the presence of MAI

In neurite outgrowth, a process that is also inhibited by the presence of MAI, addition of putrescine, a polyamine that is expressed as a consequence of CREB activation, is able to overcome the myelin mediated outgrowth block. The presence of putrescine is also required for efficient OPC differentiation. Thus it was tested whether incubation with putrescine induces OPCs differentiation in the presence of MAI. However, the results show that this is not the case and therefore the mechanisms by which CREB is able to promote OPC differentiation differ from the mechanisms by which it induces axonal outgrowth (fig. 15). Remyelination mediated by CREB thus does not act via putrescin.

### Example 11: Synergistic effects

Next it was investigated whether ROCK-II and PKC transduce inhibitory Signals via common or separate mechanisms and whether blocking both pathways at the same time could further promote OPC differentiation in the presence of myelin inhibitors. Treatment with Fasudil and Gö6976 simultaneously induced a further increase of the percentage of differentiating OPCs (fig.6; t-test: HA-1077 vs. co-incubation: p=0.0009; Gö6976 vs. co-incubation: p=0.0288). Furthermore, the treatment of OPCs on MPE with the inhibitors was able to reverse the effects of myelin on OPC morphology and resulted in the presence of more mature phenotypes (stages III and IV, fig. 6; ANOVA of early stages (I/II) in PLL, MPE, and co-treatment p=0.0027; Dunn Post test: MPE vs. co-treatment P<0.01). MPE may not include all inhibitory factors present in crude myelin preparations. To ensure that the beneficial treatment effects were not restricted to Substrates prepared with MPE OPCs plated on crude myelin preparations with HA-1077, Gö6976, or a combination of both were incubated. This resulted in a similar induction of OPC differentiation in the presence of myelin inhibitors. Taken together the present findings suggest that the inhibitory effects of myelin molecules are mediated by at least two independent pathways. Furthermore, blocking both pathways simultaneously may provide an even more potent strategy to improve OPC differentiation in an *in vivo* setting.

PKC and ROCK Inhibition have shown to exert stimulating effects on neurite outgrowth in the presence of myelin Inhibitors, and thus synergistic effects with respect to neural repair could be expected. As far as effects on other cell types are concerned, Inhibition of PKC in microglia is associated with e.g. down-regulation of MHC-II, a reduction of proinflammatory cytokine, iNOS, and TNF-alpha release in response to various inflammatory modulators including LPS, ganglioside, and IFNgamma suggesting a reduction of proinflammatory activity in microglia. On the other hand, it has been reported that PKC Inhibition lead to reduced CR3/Mac-1 and SRAI/II mediated myelin phagocytosis which may be relevant to myelin repair although the mechanisms we report aim at neutralising the effects of myelin associated Inhibitors on OPC differentiation. Astrocytes react upon PKC Inhibition by e.g. decreased activation of ATP-mediated glutamate outwards channels and TGFbl production. Furthermore, the response to LPA is altered by a reduced proproliferative intracellular Ca2+ increase and NGF secretion. It seems unlikely that the reported effects of PKC Inhibition on astrocytes will negatively impact remyelination. Under the influence of Fasudil, rat astrocytes transform into process bearing cells in vitro, which can be explained by direct effects of the Rho-cascade on the cytoskeleton

These data suggest that inhibiting PKC and/or Rho-signalling would be beneficial either acutely following demyelination, where exposure to myelin debris, that has yet to be cleared, might critically interrupt the initiation of remyelination, resulting in its long term failure or in chronic disease where OPCs remain exposed to a differentiation-inhibitory environment. The data in the current study paves the way for subsequent testing of pharmacological interventions in animal models and further clinical evaluation if the outcome were to be successful.

### Example 12: Neutralisation of Ephrin-B3 and Sema3A by antibody treatment.

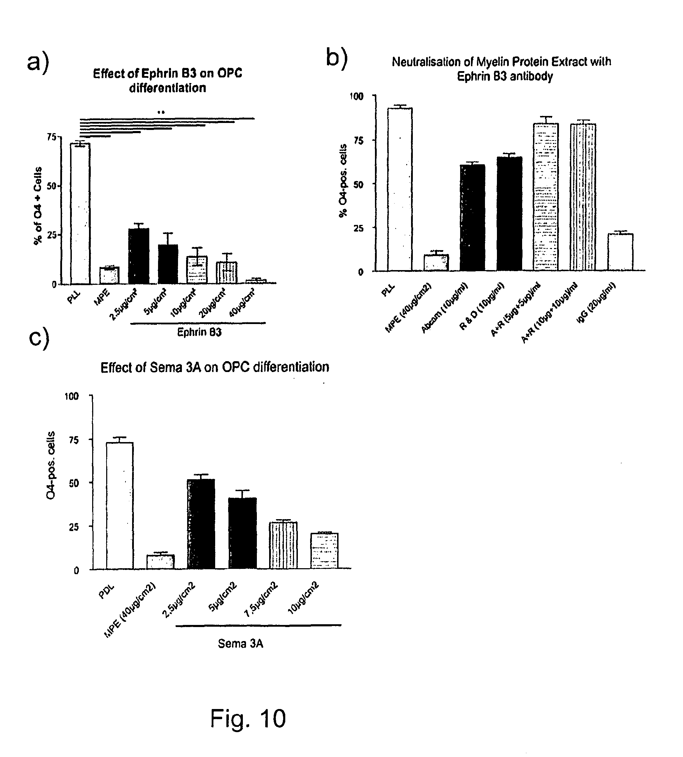

As shown in Fig. 10a and 10c, Ephrin-B3 and Sema3A are potent inhibitors of OPC differentiaten. Antibodies against these targets can neutralize the inhibitory action of a myelin protein extract on oligodentrocyte precursor cell differentiation (Fig. 10b). MPE substrates were prepared as outlined above. To neutralise inhibitory epitopes of Ephrin B3, the substrates were incubated with antibodies against Ephrin B3. Following a washing step with PBS, OPCs were plated onto the antibody-treated substrates and the percentage of differentiating 04-positive cells determined. The results demonstrate that neutralisation of OPCs with the antibodies from two manufacturers (R&D and AbCam) were able to significantly enhance the numbers of differentiating OPCs in the presence of myelin. Furthermore, a combination of the two antibodies was able to completely restore OPC differentiation in the presence of myelin inhibitors. Incubation of substrates with unspecific IgG as a control did not stimulate OPC differentiation on myelin.

## Claims

1. An agent capable of enhancing the PKA-p38-CREB pathway, suppressing the Fyn-RhoA-Rock and/or PKC-alpha-MARCKS pathways or suppressing Ephrin-B3 or Sema3A for the treatment of damaged myelin sheaths, wherein the agent neutralizes the effect of inhibitors of the regeneration of damaged myelin sheaths by oligodendrocytes and the inhibitors are selected from myelin associated inhibitors, in particular Ephrin-B3 and inhibitors of the glial scar, in particular Sema3A.

2. Agent according to claim 1 being an enhancing agent of the PKA-p38-CREB pathway, preferably selected from cAMP agonists or phosphodiesterase-4 inhibitors, PKA agonists, p38 MAPK agonists, or CREB agonists, even more preferred being selected from rolipram or db-cAMP.

3. Agent according to claim 1 being a suppressing agent of the Fyn-RhoA-Rock pathway and selected from ROCK inhibitors, Fyn agonists and, preferably being selected from RNAi of RhoA, RNAi of ROCK-II or combinations thereof.

4. Agent according to claim 1 being a suppressing agent of the Fyn-RhoA-Rock pathway, preferably being a Rho inhibitor, more preferred being Fasudil or Y-27632, for the combined administration with an anti-inflammatory agent or with an oligodendrocyte precursor cell or a stem cell differentiating into oligodendrocytes.

5. Agent according to claim 1 being a suppressing agent of the PKC-alpha-MARCKS pathway, preferably a PKC-alpha inhibitor, more preferred being selected from RNAi of PKC-alpha, Gö-6976.

6. Agent according to claim 1 being one or a combination of anti-Ephrin-B3 antibodies or one or a combination of anti-Sema3A antibodies.

7. Agent according to claim 1 to 6, **characterized in that** the agent neutralizes inhibitors of oligodendrocyte precursor cell differentiation to active oligodendrocytes.

8. Agent according to claim 1 to 7, **characterized in that** the damaged myelin sheaths are associated with axon demyelination but not of axon destruction, preferably in the case of damage by inflammation, in particular in multiple sclerosis.

9. Agent according to claim 1 to 8, **characterized in that** the damaged myelin sheaths are associated with disease or condition selected from being multiple sclerosis, stroke, nerve injury, in particular spinal cord injury, in central nervous system axons, of corticospinal tract axons, of dorsal column axons, acute disseminated encephalomyelitis, transverse myelitis, Alexander's disease, Canavan disease, Cockayne's syndrome, Pelizaeus-Merzbacher's disease, optic neuritis, hereditary leukencephalopathy, central pontine myelinosis, Deep White Matter Ischemia, Progressive Multifocal Leukoencephalopathy, Post-Infectious Encephalitis, demyelinating HIV Encephalitis, demyelinating Radiation Injury, acquired toxic-metabolic disorders, Posterior Reversible Encephalopathy Syndrome, central pontine myelinolysis, demyelinating heredetary metabolic disorders, including leukodystrophies, in particular adrenoleukodystroph, Krabbe's globoid cell, metachromatic leukodystrophy.

10. Agent according to claims 1 to 9, selected from agents capable of enhancing the PKA-p38-CREB pathway or of suppressing the Fyn-RhoA-Rock and/or PKC-alpha-MARCKS pathways or suppressing Ephrin-B3 or Sema3A as defined in the preceding claims for administration together with at least one, preferably at least two additional agent suppressing a different pathway or molecular target of the pathways or target selected from Ephrin-B3 or Sema3A, as defined in the preceding claims.

11. Agent according to claims 1 to 10 for the combined administration with an anti-inflammatory agent, preferably a corticosteroid, cortisone, mitoxantron, natalizumab, interferon beta or glatiramer acetate.

12. A kit comprising at least two pharmaceutical compounds selected from at least two different groups of the following groups:
group 1: cAMP agonists or phosphodiesterase-4 inhibitors, PKA agonists, p38 MAPK agonists or CREB agonists;
group 2: ROCK inhibitors, Fyn agonists or Rho inhibitors;
group 3: PKC-alpha inhibitors,
group 4: Ephrin-B3 inhibitors or Sema3A inhibitors.

13. A kit comprising at least one pharmaceutical compounds selected from the following groups:
group 1: cAMP agonists or phosphodiesterase-4 inhibitors, PKA agonists, p38 MAPK agonists or CREB agonists;
group 2: ROCK inhibitors, Fyn agonists or Rho inhibitors;
group 3: PKC-alpha inhibitors,
group 4: Ephrin-B3 inhibitors or Sema3A inhibitors and an anti-inflammatory agent, preferably for the combined administration for the treatment of myelin sheath damage.

14. A kit according to claim 12 or 13, comprising at least inhibitors or agonists of groups 1 and 2, of groups 2 and 3, of groups 1 and 3, or preferably of groups 1, 2 and 3.

15. A kit according to one of claims 12 to 14, comprising at least inhibitors or agonists of groups 1 and 4, of groups 2 and 4, or of groups 3 and 4.
